# EUROPEAN PATENT SPECIFICATION

(11) **EP 2 261 673 B1**
(45) Date of publication and mention of the grant of the patent: **24.01.2018**
(21) Application number: 09727772.7
(22) Date of filing: 27.03.2009
(51) Int. Cl.: G01N 33/96, G01N 21/27, G01N 33/49

(54) **METHOD FOR PREPARING CALIBRATING REAGENT, CALIBRATING REAGENT, AND METHOD FOR CALIBRATING BLOOD COMPONENT MEASURING DEVICE USING SAID CALIBRATING REAGENT**
VERFAHREN ZUR HERSTELLUNG EINES KALIBRIERREAGENS, KALIBRIERREAGENS UND VERFAHREN ZUR KALIBRIERUNG EINES MESSGERÄTS FÜR BLUTBESTANDTEILE ANHAND DIESES KALIBRIERREAGENS
PROCÉDÉ DE PRÉPARATION D'UN RÉACTIF D'ÉTALONNAGE, RÉACTIF D'ÉTALONNAGE ET PROCÉDÉ D'ÉTALONNAGE D'UN DISPOSITIF DE MESURE DES COMPOSANTS DU SANG UTILISANT LEDIT RÉACTIF D'ÉTALONNAGE

(30) Priority: 31.03.2008 JP 2008090401; 28.08.2008 JP 2008220493
(43) Date of publication of application: 15.12.2010
(73) Proprietor: Terumo Kabushiki Kaisha, Tokyo 151-0072 (JP)
(72) Inventor: UCHIDA, Takao, Ashigarakami-gun Kanagawa 259-0151 (JP); KUMAGAI, Ayumi, Nakakoma-gun Yamanashi 409-3853 (JP); NAGASAWA, Yasushi, Nakakoma-gun Yamanashi 409-3853 (JP); ARITA, Eiji, Nakakoma-gun Yamanashi 409-3853 (JP); NAKAMURA, Toshihisa, Nakakoma-gun Yamanashi 409-3853 (JP)
(74) Representative: TBK
(86) International application number: PCT/JP2009/056351
(87) International publication number: WO 2009/123069

(56) References cited:
- WO-A1-87/06343
- WO-A1-89/12827
- GB-A- 2 308 444
- GB-A- 2 385 663
- JP-A- 1 006 865
- JP-A- 8 313 532
- JP-A- 2002 146 249
- JP-A- 2005 089 737
- US-A- 5 637 505
- US-A1- 2004 180 444

## Description

### Technical field

The present invention relates to a method of adjusting a calibration reagent used in calibrating a blood component measurement device where a blood component is reacted with a chromogenic reagent and its color is measured optically (e.g., the concentration of a blood component is measured using a test paper carrying a chromogenic reagent that reacts with saidblood component), a calibration reagent, and a method of calibrating the blood component measurement device using said calibration reagent.

### Background technology

As a method of measuring a specific component of blood, it has been known that blood is absorbed into a test paper or the like impregnated with a chromogenic reagent that reacts with a specific component of blood to measure the degree of coloring of the chromogenic reagent. Also, by using the method, a blood component measurement device detects or quantifies the amount of the specific component as the measuring object from the intensity of reflection of a light by irradiating the test paper carrying the chromogenic reagent with the light of a specific wavelength (e.g., Japanese Patent Laid-Open No. 2004-347436).

In case of such a blood component measurement device, it is necessary to confirm whether the device itself or the measuring system including the test paper is measuring said specific component correctly. Various calibration reagents (sometimes referred to as standard reagents) for calibration have been developed for that purpose. More specifically, for a case of measuring glucose as said specific component, a calibration reagent has been known in which a known amount of glucose is mixed with a buffering agent, a preservative agent, a surface active agent, a surfactant etc. in water as a solvent. (Japanese Patent No. 3509864).
US2004180444 (A1) relates to an aqueous control solution for use with a spectrophotometer or photometric test strip that includes a predetermined amount of an analyte, a hydrophobic reference dye and a surfactant, wherein the aqueous control solution includes a predetermined amount of glucose, an infrared reference dye, sodium dodecyl sulfate, and the indicator dye, such as sulforhodamine B.
US5637505 (A) relates to a method of preparing dye-based reference materials useful for calibrating or qualifying instrument systems that are diagnostic spectroscopically for hemoglobin and CO-ox fractions, wherein the dye-based reference materials are non-proteinaceous and may be interpreted by CO-oximeter instrument systems as providing a spectrum substantially equivalent to the spectrum of blood. GB2308444 (A) relates to liquid control standards for use in e.g. CO-oximetry and electrolyte determinations comprising an aqueous solution, which contains an absorbance means to provide a control test which corresponds to a predetermined level of hemoglobin or hemoglobin fractions and a sufficient concentration of a polyvinylpyrrolidone (PVP) polymer or which contains a predetermined amount of electrolytes and a sufficient concentration of PVP polymer.
GB2385663 (A) relates to a method of monitoring calibration of a spectrophotometric apparatus that comprises one or more calibration algorithms for one or more analytes.

### Disclosure of the invention

However, in case of a calibration reagent for measuring a specific component contained in blood such as glucose as disclosed in Japanese patent No. 3509864, there is a problem of causing errors in an actual calibration of the device, as the color of the solution is almost transparent, distinctly different from blood color.

This problem is basically due to the fact that when a test paper impregnated with the chromogenic reagent is caused to absorb blood, it develops coloring due to the specific component as well as blood color; while in case of the calibration reagent it develops only the reaction color of the chromogenic reagent (color of the pigment generated) without blood color, so that it causes the value to shift in the same measurement algorithm by the amount of the blood color.

Therefore, it is an intention of the present invention to provide a method of adjusting a calibration reagent that allows to calibrate a measurement device measuring a color under a condition in which the blood color is mixed with the color generated by the chromogenic reagent in contact with a blood component.

It is another intention of the present invention to provide a calibration reagent that can calibrate a measurement device measuring the color under a condition in which the blood color is mixed with a color generated by the chromogenic reagent in contact with a blood component.

It is yet another intention of the present invention to provide a method of calibrating a blood component measurement device that uses a calibration reagent that can calibrate a measurement device measuring the color under a condition in which the blood color is mixed with a color generated by the chromogenic reagent in contact with a blood component.

Moreover, the inventors of the present invention have tried to simulate the color of a calibration reagent with the blood color by adding dyes to the calibration reagent in order to solve the problems described above. By taking such a try, it was possible to solve the problems to a degree. However, it was found that the added dyes presented other kinds of problems as they had physical properties different from the blood component.

In other words, a temperature compensating algorithm is typically programmed into the measurement device to compensate for the fluctuation of the measurement value depending on the temperature condition at the time of measurement of a specific component (e.g., glucose) in a blood sample using such a measurement device. This is intended to compensate for the fluctuation of measurement values caused by the measurement device due to changes in the temperature condition at the time of the measurement (measurement values normally tend to increase as the measurement temperature rise). However, the aforementioned dye added in order to simulate the color of the calibration reagent with the blood color does not necessarily show the same temperature characteristic as blood. Consequently, a problem was found that it is impossible to make an accurate calibration of the measurement device due to the difference in the temperature characteristics between blood and the calibration reagent if the algorithm programmed for blood is applied to the calibration reagent.

As a result, yet another intention of the present invention is to provide a method of adjusting the calibration reagent in order to make it possible to accurately calibrate the measurement device even when the temperature condition at the time of measurement varies.

Yet another intention of the present invention is to provide a calibration reagent to allow the measurement device to calibrate accurately even when the temperature condition at the time of measurement varies.

Yet another intention of the present invention is to provide a method of calibrating a blood component measurement device using a calibration reagent for accurately calibrating the measurement device even when the temperature condition at the time of measurement varies.

The aforementioned intentions are achieved by the following means:
(1) A method of adjusting a calibration reagent used in calibrating a blood component measurement device intended for optical measurement of a blood component using a test paper characterized in combining two or more kinds of different dyes and adjusting in such a way that the variation tendency of the reflection-absorbance depending on the measurement temperature when the measurement is carried out by spotting said calibration reagent on one surface of said test paper and irradiating the other surface with a light of a specified wavelength becomes similar to the variation tendency of the reflection-absorbance depending on the measurement temperature when measured by spotting blood instead of said calibration reagent, wherein said two or more kinds of dyes comprise 6-amino-4-hydroxy-5-((4-nitro-2-sulfophenyl)azo)-2-naphthalenesulfonic acid and Alphazurine A.
(2) The adjustment method described in (1), wherein said two or more kinds of dyes are combined in such a way as to compensate the measurement error resulting from the fact that the calibration reagent does not contain the pigment component in blood.
(3) The adjustment method described in (2) characterized in, placing the center value of the FWHM of the emission spectrum of the light emitting element at the nominal wavelength of said light emitting element used in said blood component measurement device, selecting and combining two or more kind of dyes having different inclinations of reflection-absorbance spectrum within the wavelength range of at least said FWHM having its center value at said nominal wavelength, and adjusting the calibration reagent in such a way that the resultant inclination tendency of the reflection-absorbance spectrum within said wavelength range becomes similar to that of the reflection-absorbance spectrum of blood within said wavelength range, and/or wherein said two or more kinds of dyes comprise one or more kinds of dyes selected from a group of dyes having a maximum absorption wavelength on a wavelength side shorter than 610 nm, and one or more kinds of dyes selected from a group of dyes having a maximum absorption wavelength on a wavelength side longer than 610 nm.
(4) A method of adjusting a calibration reagent used in calibrating a blood component measurement device intended for optical measurement of a blood component using a test paper characterized in combining first and second dyes having different inclinations of absorption spectrum within a wavelength range of at least the FWHM of the emission spectrum having its center value at the nominal wavelength of a light emitting element used in said blood component measurement device; and adjusting the calibration reagent in such a way that the shape of said combined absorption spectrum within said wavelength range becomes similar to the shape of the absorption spectrum of blood within said wavelength range, as well as that the variation tendency of the reflection-absorbance depending on the measurement temperature when measured by spotting said calibration reagent on one surface of said test paper and irradiating the other surface with a light of a specifiedwavelengthbecomes similar to the variation tendency of the reflection-absorbance depending on the measurement temperature when measured by spotting blood instead of said calibration reagent, wherein said first and second dyes comprise 6-amino-4-hydroxy-5-((4-nitro-2-sulfophenyl) azo) -2-naphthalenesulfonic acid and Alphazurine A.
(5) A calibration reagent used in calibrating a blood component measurement device intended for optical measurement of a blood component using a test paper characterized in that the calibration reagent comprises a known amount of the blood component, and two or more kinds of different dyes combined in such a way that the variation tendency of the reflection-absorbance depending on the measurement temperature when measured by spotting said calibration reagent on one surface of the test paper and irradiating the other surface with a light of a specified wavelength becomes similar to the variation tendency of the reflection-absorbance depending on the measurement temperature when measured by spotting blood instead of said calibration reagent, wherein said two or more kinds of dyes comprise 6-amino-4-hydroxy-5-((4-nitro-2-sulfophenyl)azo)-2-naphthalenesulfonic acid and Alphazurine A.
(6) A calibration reagent used in calibrating a blood component measurement device intended for optical measurement of a blood component using a test paper characterized in that the calibration reagent comprises a known amount of the blood component, and first and second dyes having different inclinations of absorption spectrum within a wavelength range of at least a full width at half maximum ("FWHM") of an emission spectrum having its center value of at the nominal wavelength of a light emitting element used in said blood component measurement device; and that said first and second dyes are combined in such a way that the shape of said combined absorption spectrum within said wavelength range becomes similar to the shape of the absorption spectrum of blood within said wavelength range and that the variation tendency of the reflection-absorbance depending on the measurement temperature when measured by spotting said calibration reagent on one surface of said test paper and irradiating the other surface with a light of a specified wavelength becomes similar to the variation tendency of the reflection-absorbance depending on the measurement temperature when measured by spotting blood instead of said calibration reagent, wherein said first and second dyes comprise 6-amino-4-hydroxy-5- ((4-nitro-2-sulfophenyl) azo)-2-naphthalenesulfonic acid and Alphazurine A.
(7) The calibration reagent described in any one of (5) or (6), wherein the wavelength used in measuring said blood component is the wavelength based on the chromogenic reagent that reacts with said blood component.
(8) A method of calibrating a blood component measurement device intended for optical measurement of a blood component characterized in using the calibration reagent described in any one of (5) through (7), obtaining a measurement value by applying said calibration reagent to said blood component measurement device, and determining whether or not said measurement value is within the allowable range set up from the known component amount of said calibration reagent.

The following effects can be achieved by the present invention:
According to the invention described in (1), it is possible to provide a method of adjusting the calibration reagent that can calibrate the measurement device accurately even when the temperature condition at the time of measurement varies.
According to the invention described in (2) or (3), it is possible to assimilate the color of the calibration reagent with the blood color sufficiently within the wavelength range used in the measurement even when there are other error elements.
According to the invention described in (4), it is easy to adjust the color of the calibration reagent for calibrating the device for measuring the color under the condition where the blood color and the color caused by the blood component are mixed. Moreover, it can calibrate the measurement device accurately even when the temperature condition at the time of measurement varies.
According to the invention described in (5), it is possible to provide a calibration reagent that can calibrate the measurement device accurately even when the temperature condition at the time of measurement varies.
According to the invention described in (6), in calibrating a device for measuring a color under the condition where the blood color and the color caused by blood components are mixed, it is possible to provide a calibration reagent that can execute the calibration process just like the measurement of a blood sample without using a standard curve prepared specifically for the calibration reagent, and can calibrate the measurement device accurately even when the temperature condition at the time of measurement varies.
According to the invention described in (7), it is possible to use a measuring algorithm for blood without setting up a plurality of light emitting elements since the measurement wavelength for the calibration is chosen so as to be identical to the wavelength (wavelength based on the chromogenic reagent that reacts with blood components) used for measuring the actual blood sample.
According to the invention described in (8), it is possible to measure by using the calibration reagent similarily as blood without setting up a standard curve prepared for the calibration reagent or a measuring mode for the calibration reagent additionally.

### Brief description of drawings

Fig. 1 is a diagram for describing the outline constitution of a glucose measurement device.
Fig. 2 is a diagram for describing individual difference between light emitting spectra of light emitting diodes.
Fig. 3 is a graph showing absorption spectrum of coloring by a chromogenic reagent when a known amount of glucose is added to blood and an uncolored calibration reagent and measured.
Fig. 4 is a diagram showing an absorption spectrum of Erioglaucine.
Fig. 5 is a diagram showing an absorption spectrum of Alphazurine A.
Fig. 6 is a diagram showing an absorption spectrum of Amaranth
Fig. 7 is a diagram showing an absorption spectrum of dye A.
Fig. 8 is a diagram showing an absorption spectrum of Nitro Red.
Fig. 9A is a graph showing reflectance spectra of reference example 1-1 and comparative example 1.
Fig. 9B is a graph showing absorption spectra of reference example 1-1 and comparative example 1.
Fig. 10A is a graph obtained by overlaying Fig. 9A with the emission spectra of light emitting elements.
Fig. 10B is a graph obtained by overlaying Fig. 9B with the emission spectra of light emitting elements.
Fig. 11 is a diagram obtained by overlaying the absorption spectra of reference example 1-1 and comparative example 1 with a single absorption spectrum of each of Erioglaucine (ER) and Amaranth (AM).
Fig. 12A is a graph showing reflectance spectra of example 1-2 and comparative example 1.
Fig. 12B is a graph showing absorption spectra of example 1-2 and comparative example 1.
Fig. 13A is a graph obtained by overlaying Fig. 12A with the emission spectra of light emitting elements.
Fig. 13B is a graph obtained by overlaying Fig. 12B with the emission spectra of light emitting elements.
Fig. 14 is a diagram obtained by overlaying the absorption spectra of example 1-2 and comparative example 1 with a single absorption spectrum each of Alphazurine (AL) and dye A.
Fig. 15 is a graph showing reflection-absorbance spectra of blood samples containing 180 mg/dL of glucose obtained when the measurement temperature varies in 10, 15, 20, 25 and 30°C overlaying each other.
Fig. 16 is a graph showing measurement values compensated by using a temperature compensation algorithm after glucose concentrations of blood (blood glucose level: 180 mg/dL) are measured under five different temperature conditions (10, 15, 20, 25 and 30°C).
Fig. 17 is a graph showing reflection-absorbance spectra obtained by spotting a calibration reagent added with Erioglaucine on test paper and measuring by use of a spectrometer under different temperature conditions (10, 15, 20, 25 and 30°C).
Fig. 18 is a graph showing reflection-absorbance spectra obtained by spotting a calibration reagent added with Alphazurine A on test paper and measuring by use of a spectrometer under different temperature conditions (10, 15, 20, 25 and 30°C).
Fig. 19 is a graph showing reflection-absorbance spectra obtained by spotting a calibration reagent added with dye A on test paper and measuring by use of a spectrometer under different temperature conditions (10, 15, 20, 25 and 30°C).
Fig. 20 is a graph showing reflection-absorbance spectra obtained by spotting a calibration reagent (example) added with a combination of Alphazurine A and dye A on test paper and measuring by use of a spectrometer under different temperature conditions (10, 15, 20, 25 and 30°C).
Fig. 21 is a graph obtained by plotting reflection-absorbance spectra at a wavelength of 610 nm shown in Figs. 15, 18, 19 and 20 against measurement temperatures.
Fig. 22 is a graph showing measurements obtained by calibrating by using calibration reagents added with Alphazurine A only, dye A only, or a combination of Alphazurine A and dye A, and applying the temperature compensation algorithm for blood similar to the one used in Fig. 16.
Fig. 23 is a graph showing the result of observation of the time course of reflection-absorbance obtained when the measurement temperature was varied for an aqueous solution containing Erioglaucine only.
Fig. 24 is a graph showing the result of observation of the time course of reflection-absorbance obtained when the measurement temperature was varied for an aqueous solution containing Alphazurine A only.

### Reference code

- 100: Measurement device main body
- 101: Test paper
- 102: Light emitting element
- 103: Light receiving element
- 111: Holder

### Embodiments

Various embodiments of the present invention will be described below with reference to the accompanying drawings:
The calibration reagent provided by the present invention contains a known amount of blood components and two or more kinds of different dyes. In this calibration reagent these two or more kinds of dyes are combined in such a way that the properties of the calibration reagent are assimilated with the properties of blood.

In a calibration reagent according to an embodiment of the present invention, when the center value of the FWHM of the emission spectrum of the light emitting element is placed at the nominal wavelength of the light emitting element used in the blood component measurement device, the two dyes comprises a first dye having an absorption spectrum of a higher absorbance on the longer-wavelength side than on the shorter-wavelength side relative to the nominal wavelength of the light emitting element within a wavelength range of at least the FWHM having the center value at the nominal wavelength, and a second dye having an absorption spectrum of a higher absorbance on the shorter-wavelength side than on the longer-wavelength side relative to the nominal wavelength as well.

The calibration reagent adjusted by these two dyes has the same shape of absorption spectrum as the shape of the absorption spectrum of blood within the wavelength range of the FWHM of the emission spectrum having its center at the maximum emission wavelength of the emission spectrum of the light emitting element used for the blood component measurement device.

Such a method of adjusting the absorption spectrum of the calibration reagent using the two dyes will be described in detail below.

First, the blood component measurement device for measuring specific components of blood (hereinafter it may be referred to simply as "measurement device") will be described.

The measurement device includes, for example, the blood glucose measurement device for measuring glucose in blood as disclosed in the aforementioned reference patent document 1.

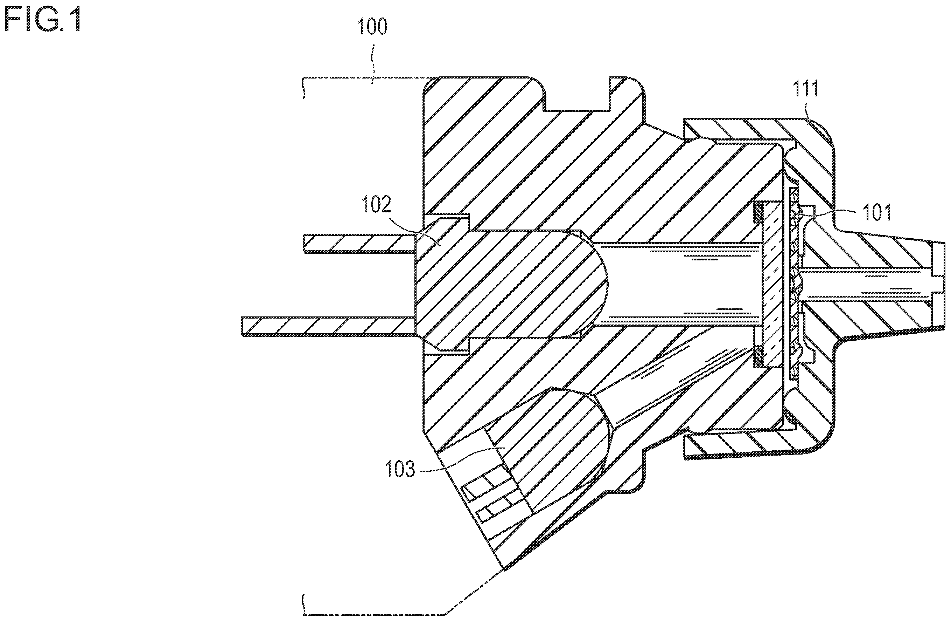

Fig. 1 is a diagram for describing the outline constitution of the glucose measurement device.

The glucose measurement device has a test paper 101 consisiting of a porous membrane, a light emitting element 102 for irradiating the test paper 101 with a light of a specific wavelength, and a light receiving element 103 for receiving the reflected light. The test paper 101 is set at a holder 111. The holder 111 is detachably attached to the measurement device main body 100. The test paper 101 is impregnated beforehand with a chromogenic reagent that forms color by reacting with glucose.

The blood, which is the target of measurement, is absorbed by the test paper 101 via a fine tube part of the holder 111 as the holder 111 is mounted on the measurement device main body 100. Thus, on the test paper 101, the chromogenic reagent impregnated reacts with glucose to generate a pigment and then a color with a color density corresponding to the glucose concentration appears.

Next, the test paper 101 is irradiated with a light with a specific wavelength by the light emitting element 102, and the reflected light is received at the light receiving element 103 and then amount of the reflected light is measured. In this case, the clock is started when the coloring starts, and the glucose concentration in the blood is calculated from the intensity of the reflected light after a certain period of time.

The measurement device main body 100 has a microcomputer (not shown) built in and the microcomputer calculates the glucose concentration by comparing the electrical signal from the light receiving element 103 and the standard curve stored in advance.

Although it is not shown, the measurement device has a display device for displaying the measurement results, an adjuster for adjusting amount of the electrical signal (gain) from the light receiving element 103, etc.

Such a measurement device selects and uses a characteristic and quantitative wavelength in the reflection-absorbance spectrum of the pigment generated when the chromogenic reagent reacts with glucose, which is the component measured, at the light emitting element 102. Therefore, a light emitting diode (LED) that emits a light of such a specific wavelength is normally used. Thus, the standard curve for obtaining the glucose concentration from the reflection light intensity (signal voltage from the light receiving element 103 in actuality) is prepared based on the specific wavelength.

The wavelength of the light emitting diode presents a bell shape curve with an expanding skirt area having the nominal wavelength as the maximum emission wavelength (peak) . However, the light emitting diode has individual differences, and its maximum emission wavelength shifts slightly from the nominal wavelength and fluctuates in the vicinity of the nominal wavelength.

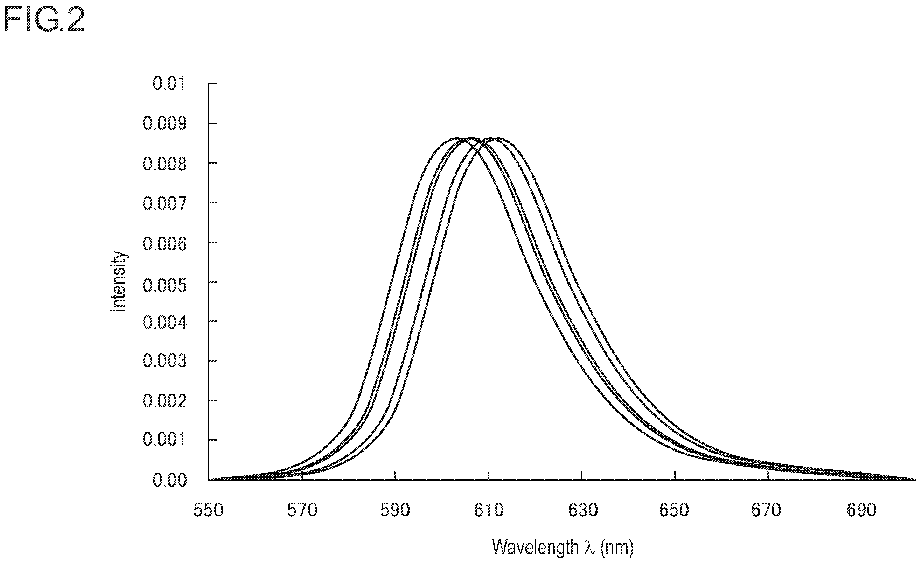

Fig. 2 is a diagram showing light emitting spectra of a plurality of light emitting diodes in order to explain individual differences of the light emitting spectra of light emitting diodes. The intensity shown in the diagram indicates the ratio for each wavelength relative to the total light released by the light emitting diode.

The data of five light emitting diodes available on the market for a nominal wavelength of 610 nm are shown here. As can be seen from the diagram, the actual maximum wavelength is shifted by approximately 5 nm above or below the center of 610 nm. In other words, the actual emission wavelengths of light emitting diodes vary to a certain degree relative to the set wavelength (nominal wavelength). Also, the spread of the emission spectrum is approximately 570 - 680 nm and the range of fluctuation of the maximum wavelength of the emission spectrum is approximately 600-615 nm.

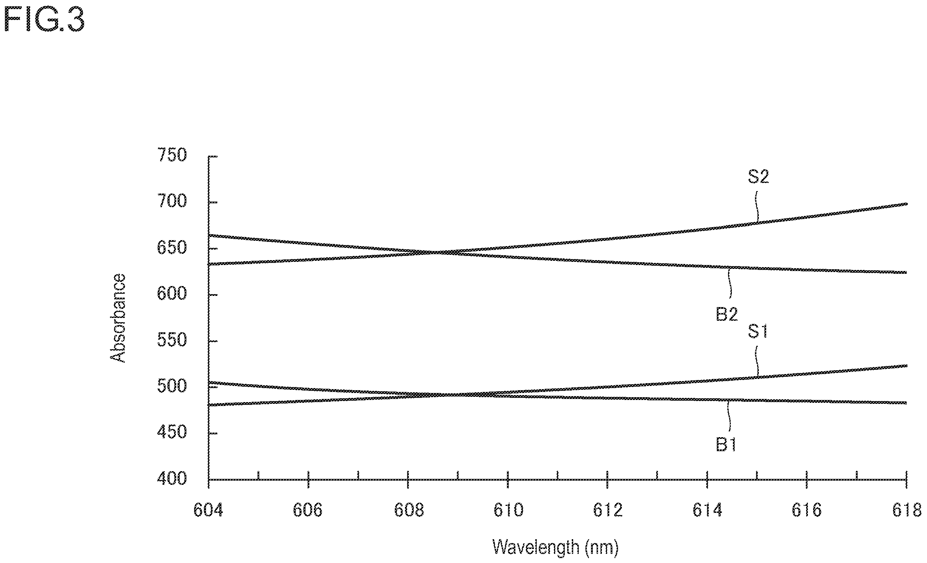

On the other hand, the absorption spectrum of blood varies with wavelengths. Fig. 3 is a graph showing the absorption spectra of blood and non-colored calibration reagent (i.e., the present invention is not applied) added respectively with known glucose amount before measurement.

Fig. 3 shows B1: blood adjusted to have glucose 70 mg/dL, S1: non-colored calibration reagent adjust to have glucose 70 mg/dL, B2: blood adjusted to have glucose 180 mg/dL, and S2: non-colored calibration reagent adjusted to have glucose 180 mg/dL, respectively.

As shown in the diagram, non-colored calibration reagents, regardless of whether glucose concentration is high or low, show lower absorbances on the side of shorter wavelengths and higher absorbances on the side of longer wavelength relative to a boundary wavelength of approximately 609 nm, compared with blood.

From this we learn that in case of blood and non-colored calibration reagent the measurement errors increase corresponding the wavelength shift when the measurement wavelength shifts. Therefore, it is necessary to separately prepare two kinds of standard curves, the standard curve to obtain the glucose concentration from the coloring in measurement of blood and the standard curve to obtain the glucose concentration from the coloring in measurement of a non-colored calibration reagent, in an actual measurement device.

In order to obtain the glucose concentration from the coloring in measuring a calibration reagent by using only the standard curve for blood without preparing those cumbersome two standard curves, the base absorption spectrum of the calibration reagent can be aligned with the absorption spectrum of the blood color. However, in order to assimilate the blood color completely, many (3, 4 or more) dyes may be required, and it is extremely difficult to adjust the mixing ratio thereof.

Therefore, in the present embodiment, it was intended to achieve an absorption spectrum having a similar shape to the absorption spectrum of blood only within a certain range centering on around the wavelength used in the measurement. If it is limited to such a certain range it is easy to match it and, moreover, if it is intended simply to match the shape of the spectrum not completely but approximately, the adjustment can be easily done by using only two dyes.

It is sufficient that the particular wavelength range is wide enough to include the differences (fluctuations) of emission spectrum generated by individual light emitting elements 102 mentioned in the above. In other words, no major measurement discrepancies with blood can be expected even if the maximum emission wavelength of the light emitting element 102 fluctuates so long as the tendencies of their absorption spectra are similar to that of the blood color within the range where the maximum emission wavelength of the light emitting element 102 fluctuates by individual light emitting element2 102.

With reference to Fig. 2 again, the fluctuations of the maximum emission wavelength of the light emitting element 102 are approximately within the range of 600 - 615 nm as mentioned above. We can see that the range of the fluctuations is, even considering a safety factor for the spread of the fluctuations, well within the range of the FWHM centering on the nominal wavelength, i.e., 610 nm, or the wavelength range of 580 - 630 nm, judging from Fig. 2. Therefore, it is sufficient that the color of the calibration reagent has an absorption spectrum of the same shape (tendency) as that of the blood color within the wavelength range of at least FWHM centering on the nominal wavelength when the center value of the FWHM of the emission spectrum of the light emitting element is placed at the nominal wavelength of the light emitting element used in the blood component measurement device. Hereinafter, the wavelength range of the FWHM of the emission spectrum of the light emitting element 102 will be referred to as "specified range."

Two kinds of dyes are used in order to match the absorption spectra within this specified range. One is a first dye with an absorption spectrum having a higher absorbance on the longer-wavelength side than on the shorter-wavelength side relative to the nominal wavelength of the emission spectrum. Another one is a second dye with an absorption spectrum having a higher absorbance on the shorter-wavelength side than on the longer-wavelength side. In other words, these two dyes have different absorption spectrum inclinations within the specified range.

Bymeans of using these two dyes, it becomes possible to adjust the liquid color having an absorption spectrum showing the same inclination tendency as the absorption spectrum of blood with the specified range. In other words, it can be achieved by increasing the quantity of the first dye if it is desired to incline the wave shape so as to elevate the longer-wavelength side of the absorption spectrum, or by increasing the quantity of the second dye if it is desired to incline the wave shape so as to elevate the shorter-wavelength side thereof. Also, if it is desired to intensify the general color density, it can be achieved by increasing the concentrations of both dyes relative to the entire solvent while maintaining the ratio between the first and second dyes that achieves the desired absorption spectrum.

The light that affects the reflectance of the test paper 101 when the reflectance of the test paper 101 is measured is not the entire wavelength range of the emission spectrum but rather the light within the range equivalent to the FWHM of the maximum emission wavelength having the strongest emission intensity. Therefore, measurement errors can be reduced sufficiently by creating an absorption spectrum having a similar inclination tendency as that of the absorption spectrum of blood within the range equivalent to the FWHM. Moreover, what is meant by "the shape is similar" is that, for example, the absorption spectrum of the calibration reagent is inclined positively within the wavelength range wherein the absorption spectrum of blood is inclined positively in the longer-range wavelength direction in a similar way (likewise in the opposite case, i.e., the absorption spectrum of the calibration reagent is inclined positively within the wavelength range wherein the absorption spectrum of blood is inclined positively in the shorter-range wavelength direction in a similar way). In other words, it can be rephrased as having the similar inclination tendency.

Such a pair of dyes can include, for example, dyes listed on Table 1, while the present invention uses Alphazurine A and 6-amino-4-hydroxy-5-((4-nitro-2-sulfophenyl) azo) -2-naphthalenesulfonic acid. Although the table shows only the maximum absorption wavelengths, specific absorption spectra of those dyes can be found in various catalogs and published documents as they are all well known dyes. One of the published documents is, for example, Sigma-Aldrich Handbook of Stains, Dyes and Indicators, Aldrich Chemical Company, 1990.

**Table 1**

| Examples of available dye | | |
|---|---|---|
| | Dye name | Maximum absorption wavelength (nm) |
| Red group | Amaranth | 521 |
| | Acid fuchsin | 540 |
| | Acid orange | 450 |
| | Acid red 88 | 510 |
| | Azocarmine G | 520 |
| | Bordeaux R | 520 |
| | Bromopyrogallol red | 550 |
| | Chromoxane cyanine R | 520 |
| | Disperse red 13 | 520 |
| | Disperse red 19 | 510 |
| | Violamine R | 530 |
| | Nitro red | 546 |
| | Sulfo rhodamine B | 560 |
| | Reactive black | 597 |
| Blue group | Erioglaucine | 625 |
| | Alphazurine A | 637 |
| | Acid blue 40 | 610 |
| | Acid blue 129 | 629 |
| | Acid green 25 | 642 |
| | Azure A | 633 |
| | Azure B eosinate | 636 |
| | Azure B tetrafluoroborate | 639 |
| | Azure C | 616 |
| | Azure II | 657 |
| | Basic blue 3 | 654 |
| | Brilliant blue G | 610 |
| | Bromothymol blue | 615 |
| | Chicago sky blue 6B | 618 |
| | Evans blue | 611 |
| | Fast green fcf | 624 |
| | Guinea green B | 618 |
| | Light green sf yellowish | 630 |
| | Lissamine green B | 633 |
| | Patent blue VF | 635 |

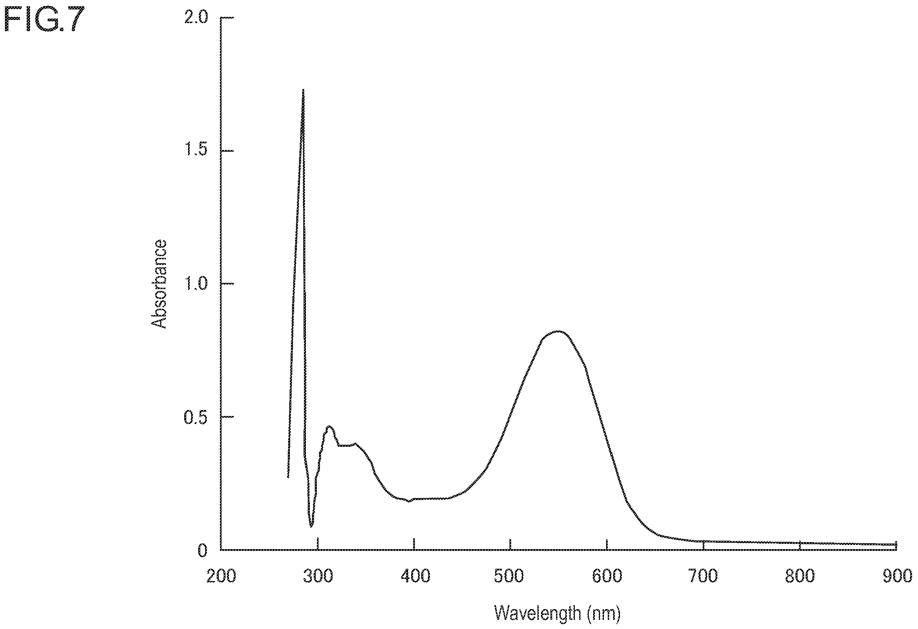

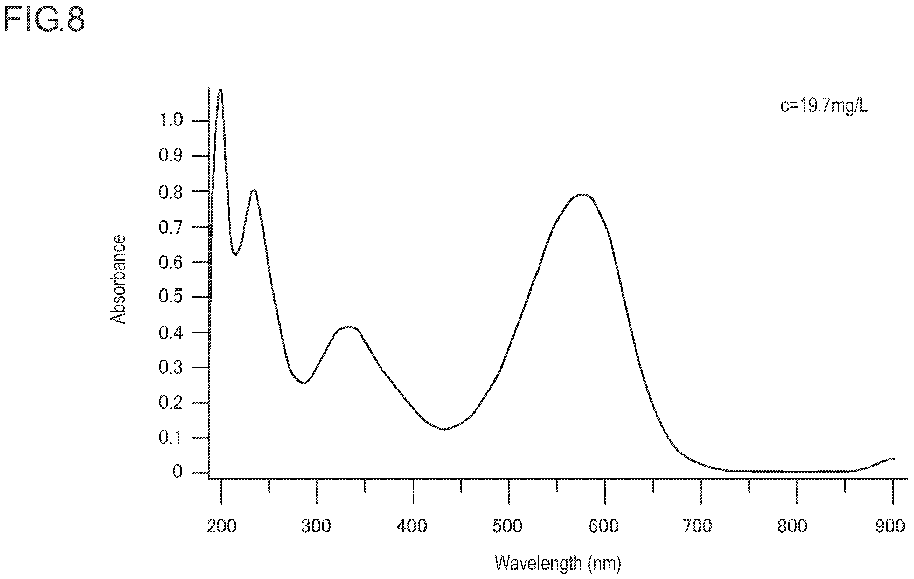

Also, as mentioned in the example later, 6-amino-4-hydroxy-5-((4-nitro-2-sulfophenyl)azo)-2-naphthalenesulfonic acid) (CAS No. 106579-09-3), which is a compound (dye) having wavelength spectrum similar to the absorption spectrum of Nitro Red, is effective. This dye is called hereunder as "dye A". Fig. 7 shows the absorption spectrum of this dye A (refer to the example to be described later). Fig 8 also shows the absorption spectrum of Nitro Red.

In another embodiment to which the present invention is applied, the calibration reagent is used for calibrating a blood component measurement device. This blood component measurement device measures the concentration of a blood component by using a test paper that carries a chromogenic reagent that reacts with the blood component. Since the detail of this device has been described above with reference to Fig. 1, it is not repeated here.

The calibration reagent of this embodiment contains the two dyes in an aqueous solution. The two dyes are combined in such a way that the variation tendency of the reflection-absorbance depending on the measurement temperature when measured by spotting the calibration reagent on one surface of the test paper provided on the aforementioned blood component measurement device and irradiating the other surface of the test paper with a light of the specified wavelength, becomes similar to the variation tendency of the reflection-absorbance depending on the measurement temperature when measured by spotting blood instead of the calibration reagent on the test paper.

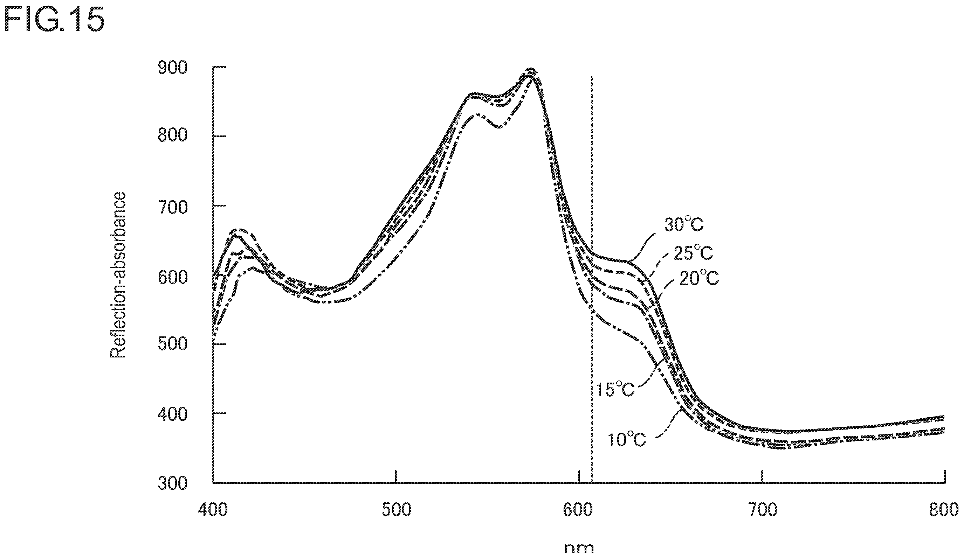

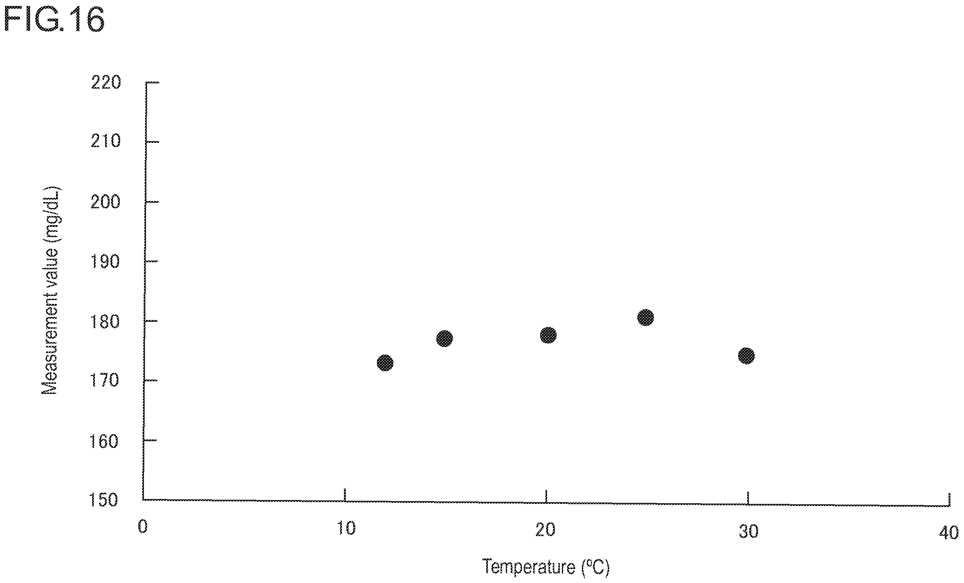

A temperature compensating algorithm is programmed for compensating the fluctuation of the measurement value due to the temperature condition during the measurement of glucose in the blood sample with the measurement device in the microcomputer (not shown) built into the measurement device main body 100 shown in Fig. 1. This makes it possible to suppress the fluctuation of the measurement value due to the temperature variation at the time of the measurement. This will be described below with reference to Fig. 15 and Fig. 16 (for details of measurement condition, etc., refer to the embodiment described later). Fig. 15 is a graph showing reflection-absorbance spectra of blood samples containing 180 mg/dL of glucose obtained when the measurement temperature varies in 10, 15, 20, 25 and 30°C overlaying each other. As shown in Fig. 15, the reflection-absorbance of the blood sample tends to shift generally in the direction of increase of the absorbance as the measurement temperature increases. Although the mechanism of absorbance changes dependent on temperature has not been made completely clear, but it is suspected that the physical properties such as spreading, infiltrating and adsorbing characteristics inside the test paper change similarly due to temperature change.

While LEDs that generate light of a wavelength of 610 nm are used for the measurement of reflection-absorbance, the change of the reflection-absorbance at the wavelength of 610 nm depending on the temperature is remarkable as shown in Fig. 15. The aforementioned temperature compensation algorithm is programmed in order to compensate the change of absorbance depending on temperature so that a constant value is displayed as the measurement value all the time. For the reference purpose, Fig. 16 is shown here as a graph showing glucose concentration of blood (blood glucose level: 180 mg/dL) measured under five different temperature conditions (10, 15, 20, 25 and 30°C) and compensated by using the temperature compensation algorithm. As can be seen from Fig. 16, the change of reflection-absorbance due to temperature change shown in Fig. 15 is compensated by applying this algorithm so that a nearly accurate and precise value is obtained as a measurement data regardless of the temperature change.

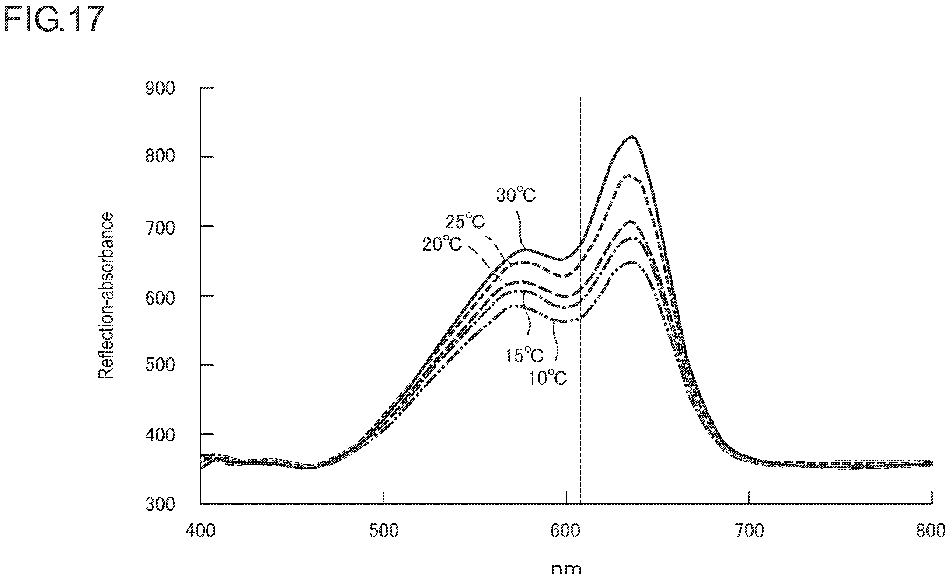

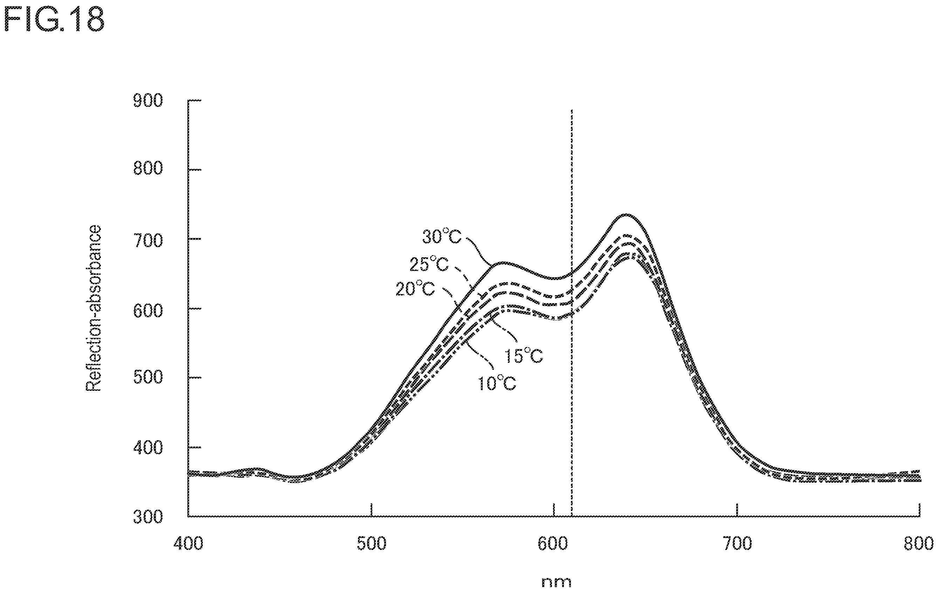

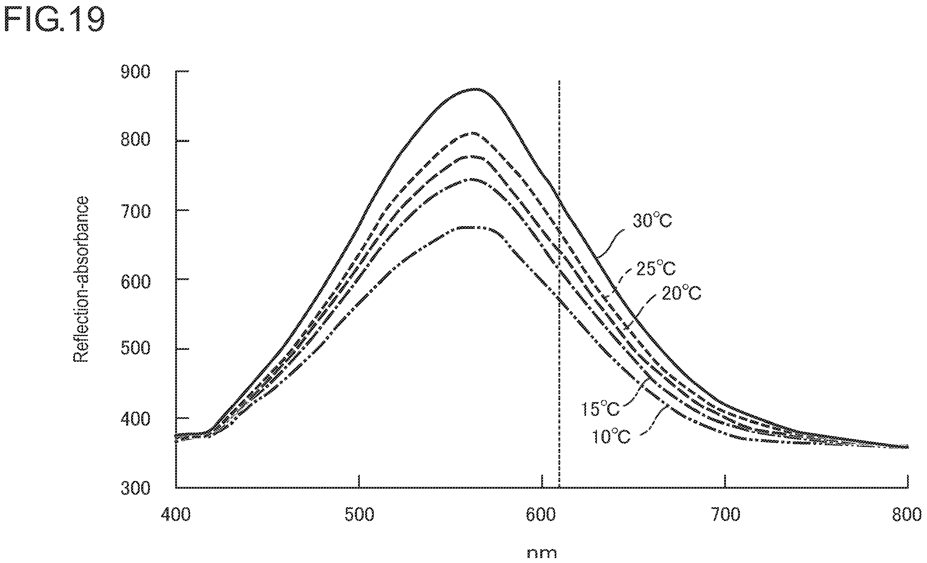

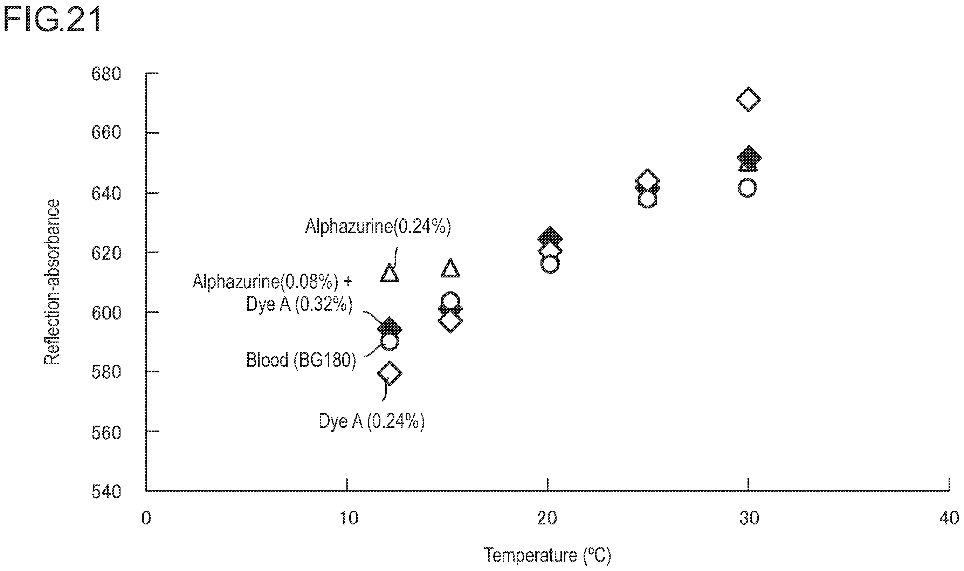

Next, as we had focused on the calibration reagent, it has been found that the dye added to the calibration reagent in order to assimilate its color with the blood color does not necessarily have a similar temperature characteristic to that of blood. This will be described with reference to the accompanying drawings. Fig. 17 - Fig. 19 are the graphs showing the reflection-absorbance spectra at various temperatures (10, 15, 20, 25 and 30°C) obtained by spotting on the test paper the calibration reagents each added with Erioglaucine (Fig. 17), Alphazurine A (Fig. 17) or the dye A (Fig. 19) respectively, and measuring with a spectrometer. As is clear from comparison between Fig. 17 - Fig. 19 and Fig. 15, the temperature characteristics at the wavelength of 610 nm are substantially different between the calibration reagents added with each dye independently (Fig. 17 - Fig. 19) and blood (Fig. 15). More specifically, in case of calibration reagents added with Erioglaucine (Fig. 17) or dye A (Fig. 19) independently, the fluctuations of the reflection-absorbance at the wavelength of 610 nm at 10 - 30°C are more than in case of blood (Fig. 15) . In other words, the inclination of the graph plotted on a coordinate system having temperature on the horizontal axis and reflection-absorbance (610 nm) on the vertical axis is larger for one added with Erioglaucine or dye A than that of blood (Fig. 21) . On the other hand, in case of the calibration reagent added with Alphazurine A (Fig. 18) independently, the inclination of the reflection-absorbance graph at the wavelength of 610 nm at 10 - 30°C is smaller than in the case of blood (Fig. 15). In other words, the inclination of the graph plotted on a coordinate system having temperature on the horizontal axis and reflection-absorbance (610 nm) on the vertical axis is smaller for a case added with Alphazurine A than in the case of blood (Fig. 21).

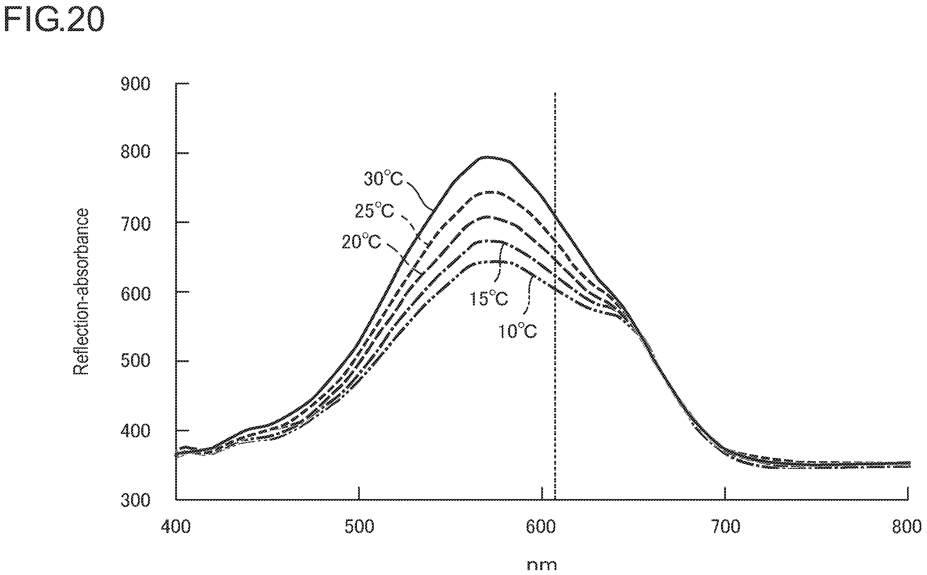

As opposed to that, the calibration reagent of the present embodiment is an aqueous solution where two dyes (Alphazurine A and dye A) are dissolved, and can solve the above problem that could not be achieved by singly adding the specific dye to the calibration reagent. Fig. 20 is a graph showing reflection-absorbance spectra under different temperature conditions of the measurements (10, 15, 20, 25 and 30°C), measured by spotting a calibration reagent added with a combination of Alphazurine A and dye A on the test paper and using a spectrometer. Fig. 21 is a graph obtained by plotting reflection-absorbances at a wavelength of 610 nm shown in Figs. 15, 18, 19 and 20 against measurement temperatures. As can be seen from these, the variation tendency depending on measurement temperature in the vicinities of the measurement wavelength obtained by spotting the calibration reagent having Alphazurine A and the dye A in combination on the test paper and measuring the reflection-absorbance becomes similar to the variation tendency depending on measurement temperature in the vicinities of the measurement wavelength measured by spotting blood instead of the calibration reagent. In other words, the combination of Alphazurine A and the dye A makes it possible to become almost completely similar to the temperature characteristic of blood.

Further, "vicinities of the measurement wavelength" means a range of ±30 nm relative to the measurement wavelength (e.g., 610 nm) in this specification. Also, above two variation tendencies "become similar" means that the output graphs for the temperature change become similar in the vicinities of the aforementioned measurement wavelength.

In adjusting the calibration reagent, as the specific form of the two kinds of dyes which the calibration reagent contains, the combination can be arbitrary so long as the two variation tendencies described above become similar , and no limitation applies to the kind or amount of dyes to be added. A person skilled in the art shall be able to select appropriately the combination of dyes by using the reflection-absorbance spectra shown in Fig. 17 - Fig. 19 as a reference. As a more specific example, first, two dyes having maximum absorbance wavelengths in the vicinity of 610 nm if the measurement wavelength is 610 nm are selected. For example, one dye is selected from Erioglaucine, Alphazurine A or the like having maximum absorbance wavelength on the longer-wavelength side of 610 nm, and another dye is selected from Nitro Red, the dye A, Reactive Black or the like having maximum absorbance wavelength on the shorter-wavelength side of 610 nm. Next, prepared are several patterns (e.g., 3 patterns) of aqueous solutions having a different ratio of concentrations of the selected two dyes. For each of them, one having the temperature characteristic similar to that of blood according to the technique shown on the section of the Examples to be described later may be selected to finish the adjustment of the calibration reagents. If possible, as shown in the more preferable embodiment described later, it is more preferable to select a dye having the inclination tendency of the reflection-absorbance spectrum in the vicinity of the measurement wavelength (610 nm) similar to that of blood under an arbitrary temperature condition. Examples of dyes that can be used include dyes shown in Table 1 described above. The aforementioned dye A can also be used preferably in the present embodiment.

The combination of two kinds of dyes to be added to the calibration reagent in the present invention is the combination of Alphazurine A and the dye A described above.

In the present embodiment, in addition to the action and effect described above, the fact that the calibration reagent contains two kinds of dyes of Alphazurine A as well as the dye A provides an advantage of compensating the measurement errors due to the fact that the calibration reagent does not contain pigment components in blood (blood pigments). Since a detailed description as to this point was made in the section of the embodiment above, further description is omitted here.

In order to calibrate the aforementioned measurement device by using the calibration reagent adjusted as described above, it is sufficient to drop the calibration reagent colored in accordance with the present embodiment on the test paper 101, and measure with the measurement device in the same way as in the normal measurement using blood. If the measurement value falls in the allowable range specified in accordance with the known glucose concentration containing in the calibration reagent, it is judged that the measurement device is acceptable from the operational standpoint. If the measurement value does not fall in the allowable range, it is judged that the measurement device is not acceptable for the measurement, or requires an adjustment and a recalibration.

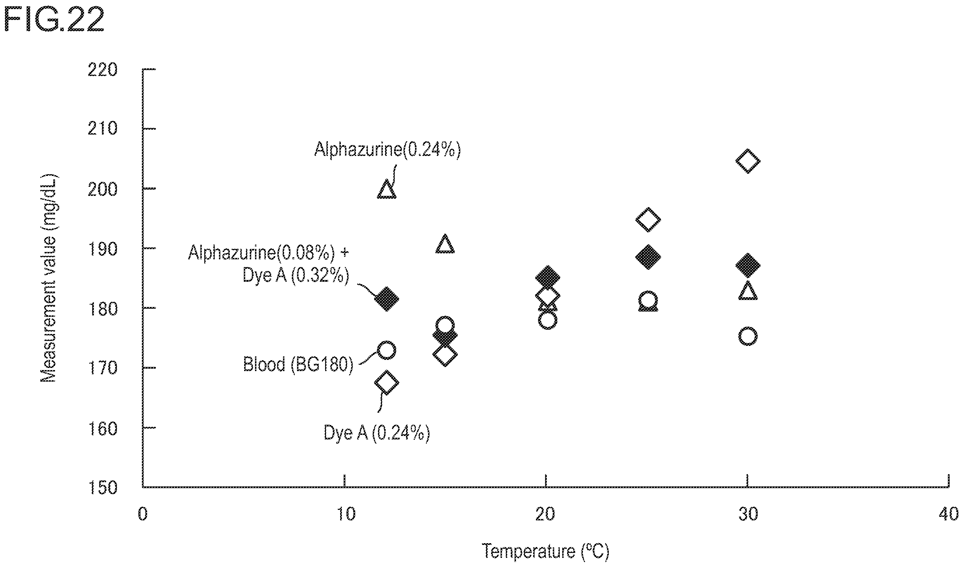

Fig. 22 shows the result of applying the calibration reagent of the present invention to a measurement device. Fig. 22 is a graph showing measurement values obtained by calibrating with the calibration reagents added with Alphazurine A only, dye A only, or a combination of Alphazurine A and dye A, and then applying the temperature compensation algorithm for blood similar to the one described above. As shown in Fig. 22, the graph of the reflection-absorbance in the vicinity of the measurement wavelength depending on the measurement temperature, which is obtained by spotting the calibration reagent on one surface of the test paper provided on the blood component measurement device and measuring the reflection-absorbance by irradiating the other surface of the test paper with a light of the specifiedwavelength, becomes quite similar to the graph of the reflection-absorbance in the vicinity of the measurement wavelength depending on the measurement temperature, measured by spotting on the test paper blood instead of the calibration reagent.

Although the measurement of glucose was described in the above as an embodiment of the present invention, the present invention is not limited to a calibration reagent used in such a measurement. For example, it can be used for adjusting the calibration reagent for the device used for measuring various blood components such as cholesterol, uric acid, pyruvic acid, etc.

Furthermore, although exemplified was a device wherein a test paper impregnated with a chromogenic reagent is used and the color forming of the test paper is measured as the reflectance ratio in the above embodiment, the present invention is not limited thereto but rather can be applied to the calibration reagent used for calibrating a device wherein a blood sample is added to a solution containing a chromogenic reagent stored in a test tube and the like to measure the transmissive-absorbance of the liquid.

### Examples

The examples of the present invention will be described below based on specific experimental examples.

### Example 1

In this example, the adjustment of the color of a calibration reagent was conducted by using two dyes, that is, Erioglaucine or Alphazurine A as the first dye, and Amaranth or the dye A as the second dye.

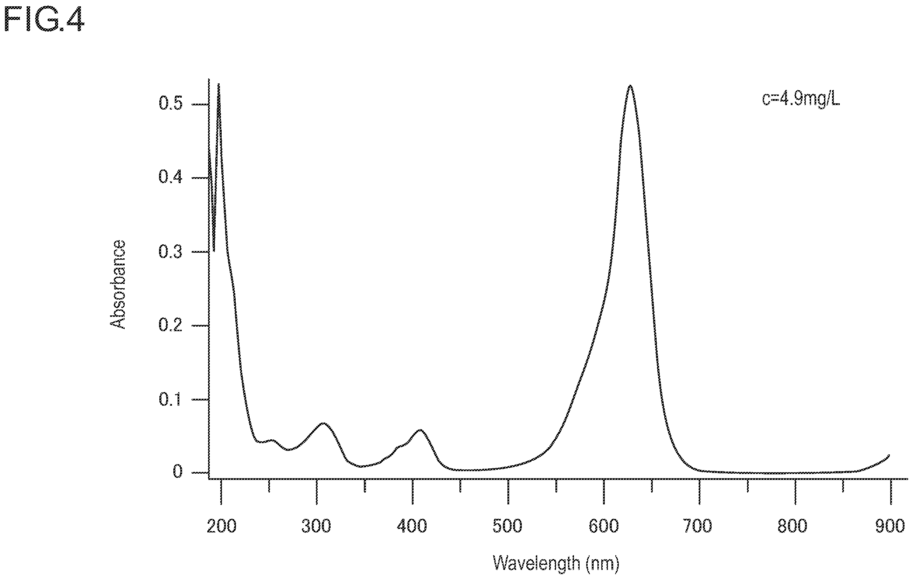

Fig. 4 is a diagram showing an absorption spectrum of Erioglaucine. As can be seen from Fig. 4, the absorption spectrum of Erioglaucine has the maximum wavelength (in water) of 625 nm, so that the addition of it increases the inclination of the absorption spectrum on the longer-wavelength side within the wavelength range of approximately 590 - 630 nm. The absorbance here means transmitted absorbance and is expressed by the logarithm of the inverse of the transmittance.

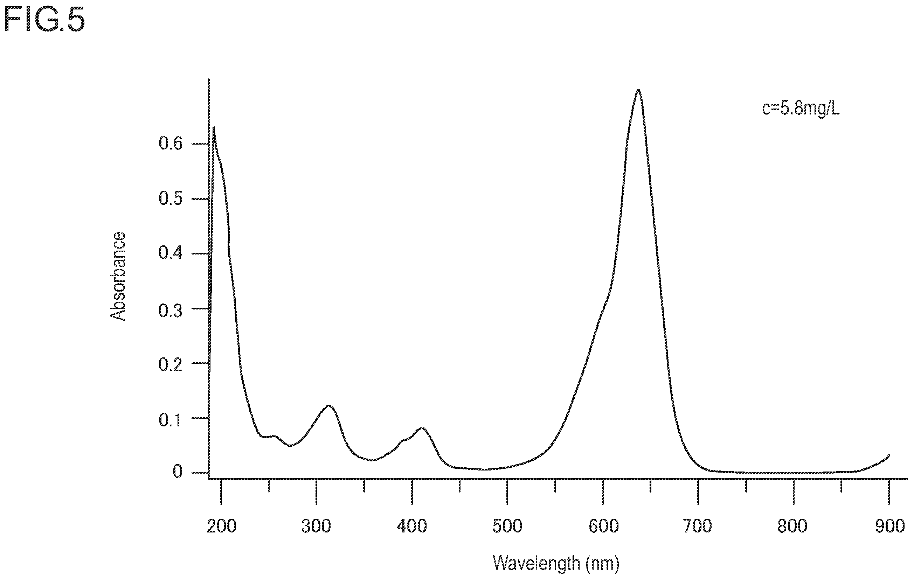

Fig. 5 is a diagram showing an absorption spectrum of Alphazurine A. As can be seen from Fig. 5, the absorption spectrum of Alphazurine A has the maximum wavelength (in water) of 637 nm, so that the addition of it increases the inclination of the absorption spectrum on the longer-wavelength side within the wavelength range of approximately 590 - 630 nm.

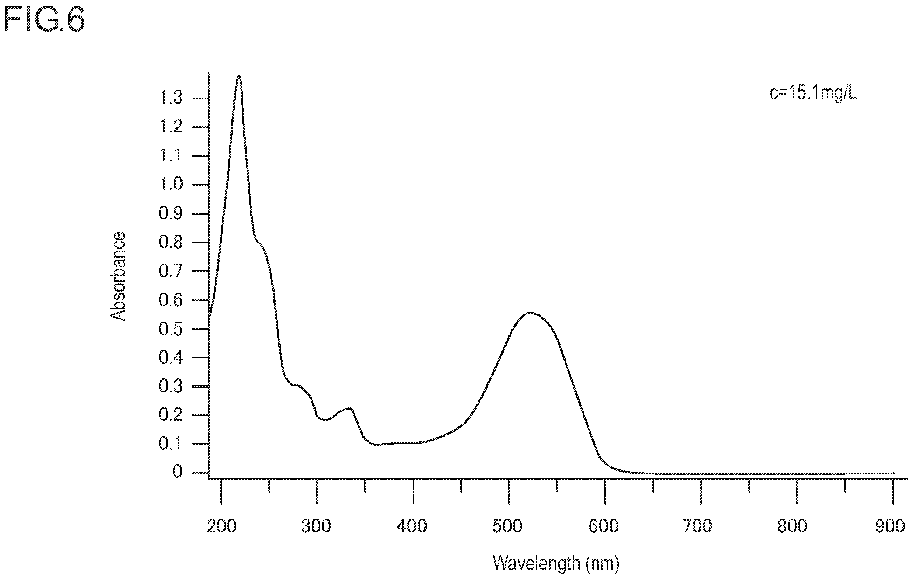

Fig. 6 is a diagram showing an absorption spectrum of Amaranth. As can be seen from Fig. 6, the absorption spectrum of Amaranth has the maximum wavelength (in water) of 521 nm, so that the addition of it increases the inclination of the absorption spectrum on the shorter-wavelength side within the wavelength range of approximately 590 - 630 nm.

Fig. 7 is a diagram showing an absorption spectrum of the dye A. As can be seen from Fig. 7, the absorption spectrum of the dye A has the maximum wavelength (in water) of 552 nm, so that the addition of it increases the inclination of the absorption spectrum on the shorter-wavelength side within the wavelength range of approximately 590 - 630 nm.

The absorption spectra shown in Figs. 4 - 6 and Fig. 8 are written on Sigma-Aldrich Handbook of Stains, Dyes and Indicators, Aldrich Chemical Company, published in 1990.

The samples are the blood sample (comparative example 1) and calibration reagent samples (examples 1-1 and 1-2) to which the present invention is applied to be colored by two dyes.

The blood sample of comparative example 1 is blood whose glucose concentration is confirmed to be 167 mg/dl by a separate measurement.

The calibration reagent sample of reference example 1-1 is prepared by adding 0.046 % by mass of Erioglaucine and 0.50 % by mass of Amaranth to pure water.

The calibration reagent sample of example 1-2 is prepared by adding 0.055 % by mass of Alphazurine and 0.23 % by mass of the dye A to pure water.

Moreover, in both reference example 1-1 and example 1-2 170 mg/dl of glucose were added. In addition to glucose, 0.73 % by mass of viscosity adjusting agent (carboxymethylcellulose (CMC 1260, made by Daicel Chemical Industries, Ltd.)), 250 mM of pH buffer (piperazine-1, 4-bis (2-ethanesulfonic acid) (PIPES, made by Dojindo Laboratories)), and 0.05 % by mass of preservative agent (sodium azide) were added as additives.

The coloring test was executed by dropping the samples of example 1 and comparative example 1 respectively on the test paper impregnated with the chromogenic reagent, and measuring the reflectance with a spectrometer USB-2000 (Pj-1181/Ocean Optics) when sufficient coloring was obtained (e.g., 10 seconds after dropping the samples). The absorbance is obtained from this reflectance and is a value calculated as 256/reflectance. The value of 256/reflectance is arbitrarily and conveniently defined to obtain absorbance from reflectance and it is not according to any regulation.

The chromogenic reagent used here is a mixture of glucose oxydase (GOD), peroxidase (POD), and 4-aminoantipyrine, and N-ethyl-N-(2-hydroxy-3-sulfopropyl)-m-toluidine.

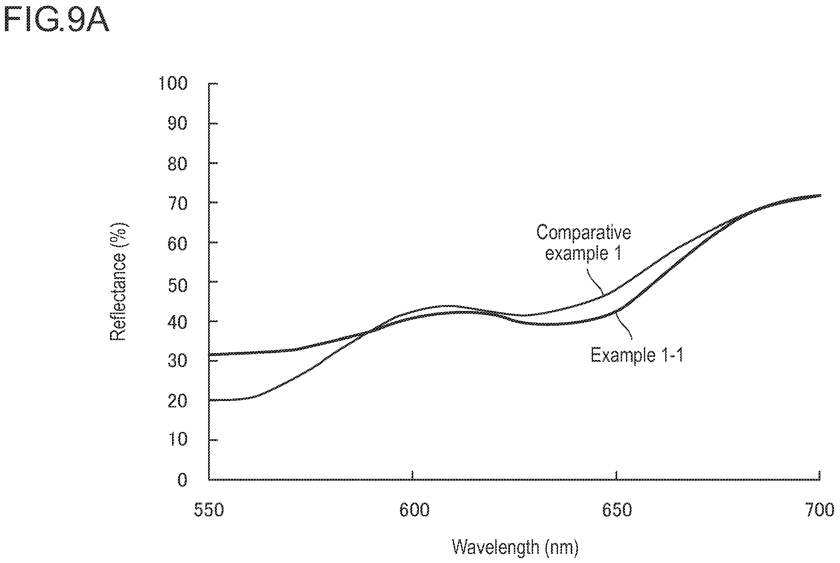

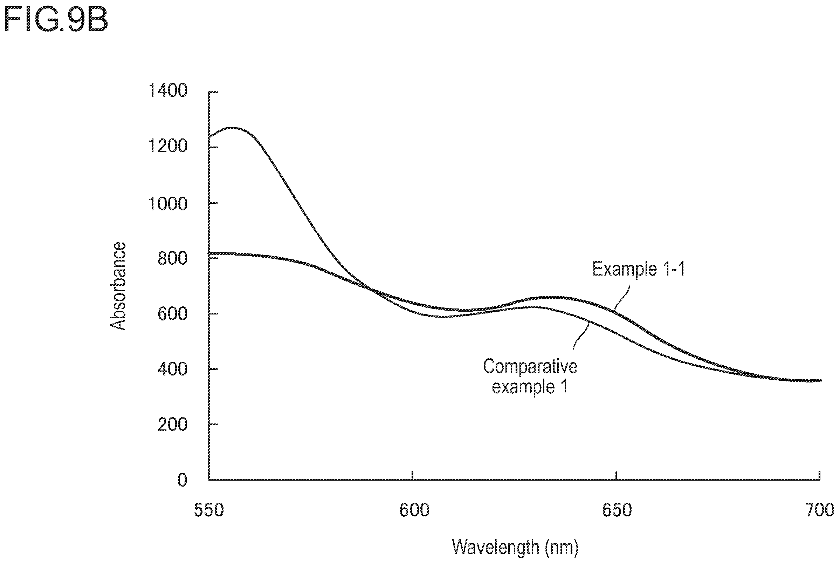

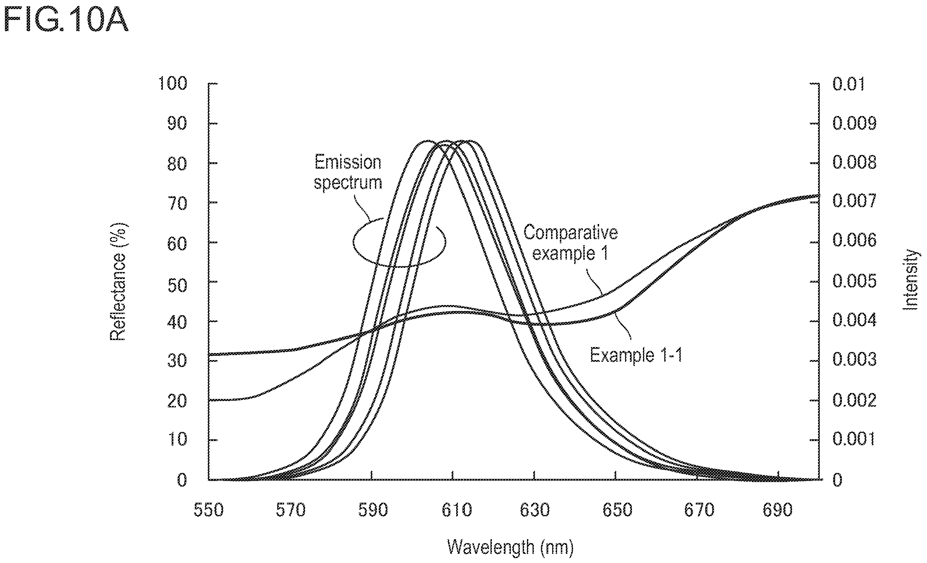

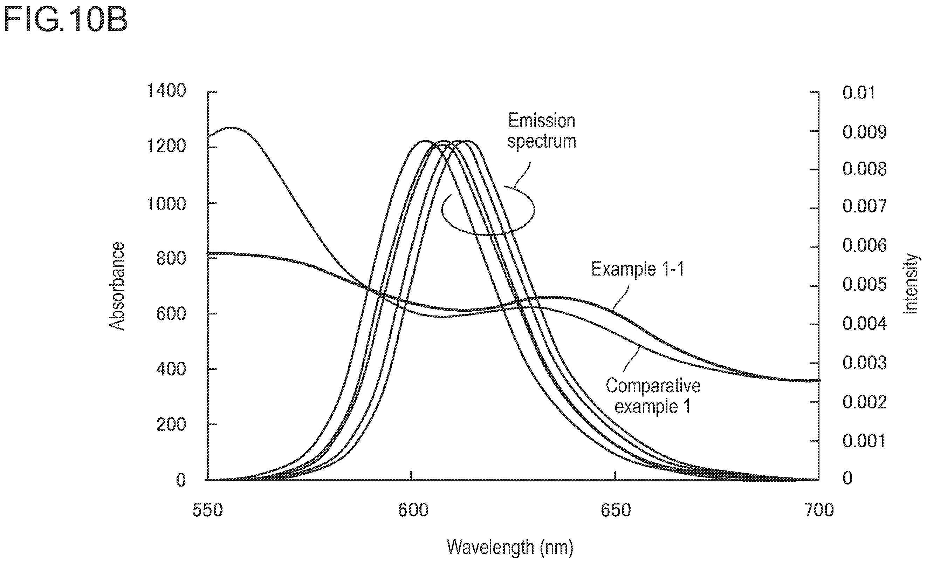

Fig. 9A is a diagram showing the graphs of reflectance spectra of reference example 1-1 and comparative example 1, while Fig. 9B is a diagram showing the absorption spectra of reference example 1-1 and comparative example 1. Fig. 10A and Fig. 10B are graphs obtained by overlaying the reflectance spectra shown on Fig. 9A and the absorbance spectra shown on Fig. 9B with the emission spectrum of the light emitting element shown in Fig. 2 respectively. The right side scale of Fig. 10 represents the emission spectrum intensity.

As can be seen from Fig. 9A and 9B, the spectrum of the calibration reagent according to example 1 is matched well with the spectrum of the blood sample (comparative example 1) in the wavelength range of approximately 570 - 650 nm including the wavelength of 610 nm characteristic of the generating pigment used in measuring the glucose concentration. In other words, the spectrum of reference example 1-1 and the spectrum of comparative example 1 have almost same inclination direction and angle in the wavelength range of approximately 570 - 650 nm.

Moreover, it is clear that the matched wavelength range is a range that generally includes the wavelength range of the fluctuating emission spectrum of the light emitting element as shown in Fig. 10A and 10B.

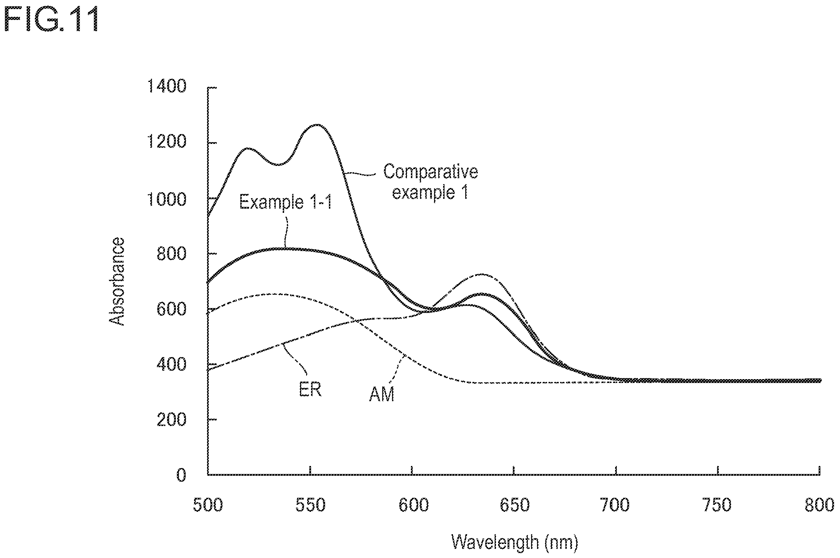

Furthermore, Fig. 11 is a diagram obtained by overlaying the absorption spectra of reference example 1-1 and comparative 1 with a single absorption spectrum of each of Erioglaucine (ER) and Amaranth (AM). This diagram also shows a wavelength range wider than those shown in Fig. 9A and 9B as well as Fig. 10A and 10B. As can be seen from this diagram, it is difficult to obtain a match with the absorption spectrum of the blood sample (comparative example 1) if a single dye is used, while, as shown in reference example 1-1, a good match with the absorption spectrum of the blood sample can be achieved within the wavelength range of the emission spectrum if these two dyes are used in combination.

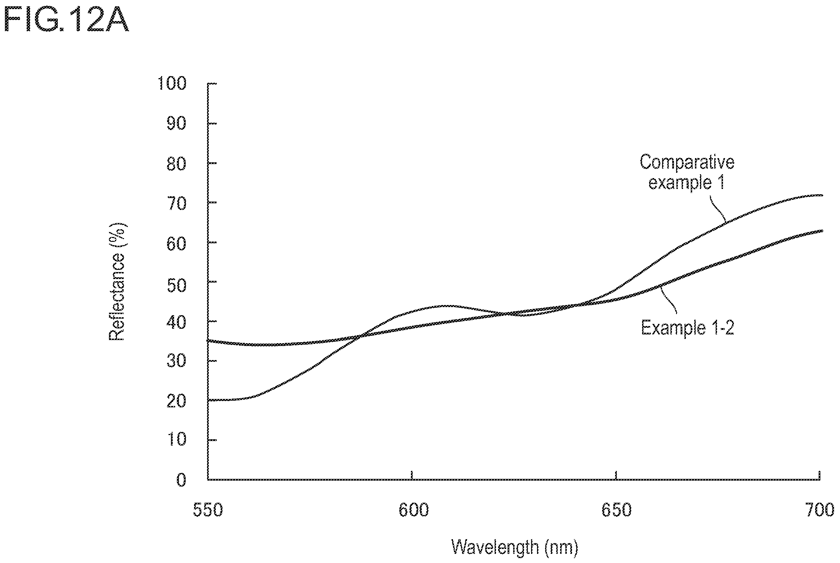

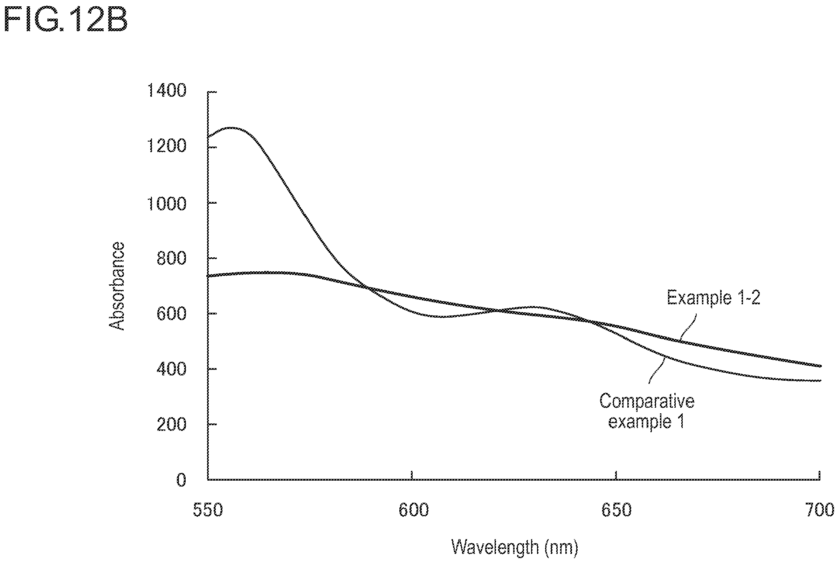

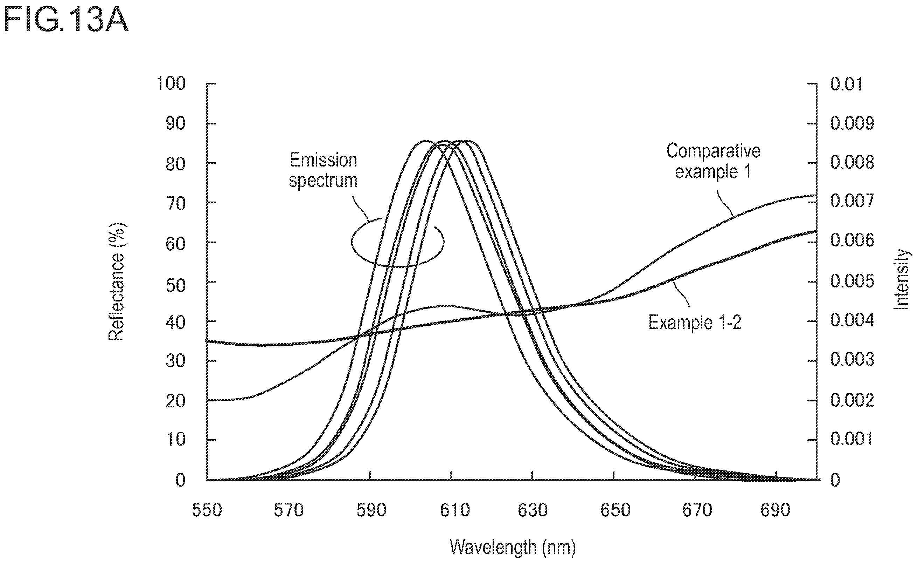

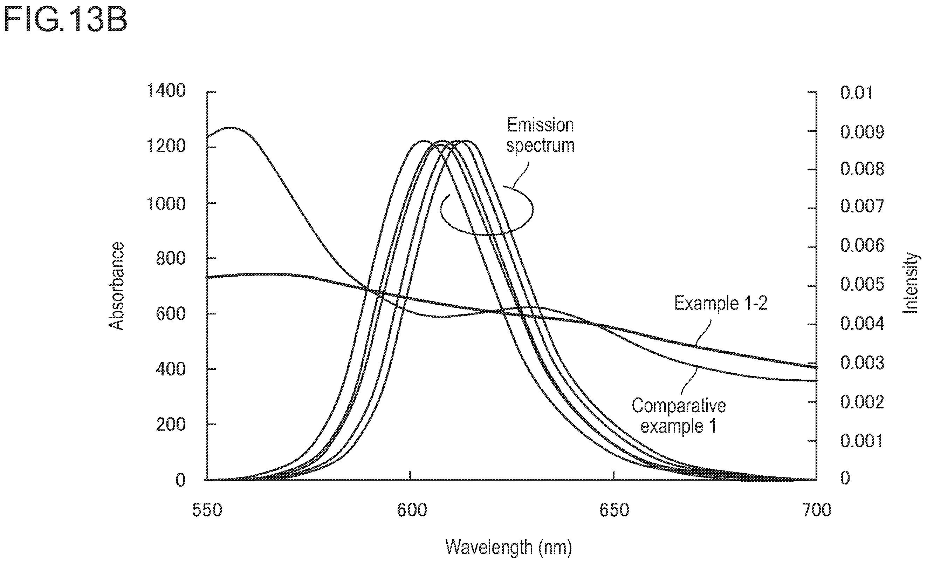

Next, Fig. 12A is a graph showing the reflectance spectra of example 1-2 and comparative example 1, while Fig. 12B shows the absorption spectra of example 1-2 and comparative example 1. Also, Fig. 13A and Fig. 13B are graphs obtained by overlaying the reflectance spectra of Fig. 12A and the absorbance spectra of Fig. 12B with the emission spectrum of the light emitting element shown in Fig. 2 respectively. The right side scale in Fig. 13A and 13B represents the emission spectrum intensity.

It is clear from Fig. 12A and 12B that the spectrum of example 1-2 is also matched well with the spectrum of the blood sample of comparative example 1 in the wavelength range of approximately 570 - 650 nm. In other words, the spectrum of example 1-2 and the spectrum of comparative example 1 have almost same inclination direction and angle in the wavelength range of approximately 570 - 650 nm.

Moreover, it is obvious that the range is a range that includes the range of the emission spectrum of the light emitting element having fluctuations as shown in Fig. 13A and 13B.

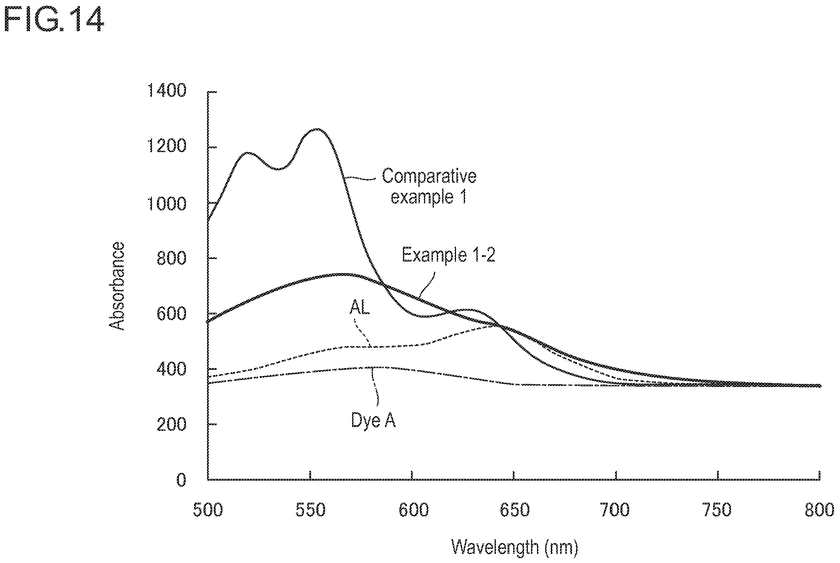

Furthermore, Fig. 14 is a diagram obtained by overlaying the absorption spectra of example 1-2 and comparative example 1 with a single absorption spectrum of each of Alphazurine A (AL) and the dye A. This diagram also shows a wavelength range wider than those shown in Fig. 12A and 12B as well as Fig. 13A and 13B. As can be seen from this diagram, it is difficult to obtain a match with the absorption spectrum (comparative example 1) of the blood sample when a single dye is used, while, as shown in example 1-2, when the two dyes are used in combination, a good match between the combined spectrum and the absorption spectrum of the blood sample can be achieved within the wavelength range of the emission spectrum.

From the results of these examples, it is learned that the coloring by using only two dyes makes it possible that the reflectance spectrum of the calibration reagent can have an similar coloring tendency to that of the reflectance spectrum in blood within the wavelength range required in measuring the glucose concentration of blood. Moreover, since a dye having a coloring tendency that shows a higher absorbance on the longer-wavelength side relative to the measurement wavelength (first dye) and another dye having a coloring tendency that shows a higher absorbance on the shorter wavelength side (second dye) are used as the two dyes, one only needs to increase the amount of the first dye if it is desired to increase the absorbance of the longer-wavelength side or increase the amount of the second dye if it is desired to increase the absorbance of the shorter-wavelength side. Thus it is easy to match the intended coloring degree.

As described in the above, in the present embodiment and example, since the two dyes are used for coloring in such a way that the absorption spectrum has the similar shape to the absorption spectrum of blood within at least the wavelength range of the FWHM centering on the maximum emission wavelength in the emission spectrum of the light emitting element used for the measurement, the device calibration can be executed accurately even when the calibration reagent is used. In particular, when the maximum emission wavelength of each light emitting element fluctuates, the device calibration can be executed without considering the fluctuations. Also, it makes it possible to calibrate the measurement device by using the standard curve prepared for blood sample measurement as it is.

In particular, by arranging in such a way that the absorption spectrum has the same shape as the absorption spectrum of blood within at least the wavelength range of 580 - 630 nm, which is the wavelength range of the FWHM centering on the nominal emission wavelength of the emission spectrum, an accurate calibration can be executed in glucose measurement.

Also, as to adjustment of a calibration reagent, the calibration reagent for calibrating a device used for glucose measurement can be easily adjusted by using a first dye having an absorption spectrum peak within a wavelength range not less than 610 nm and less than 700 nm and a second dye having an absorption spectrum peak within a wavelength range not less than 560 nm and less than 610 nm.

Although the measurement of glucose was described in the above as an example of the present invention, the present invention is not limited to the calibration reagent used in such a measurement.

For example, it can be used for adjusting the calibration reagent of the device used for measuring various blood components such as cholesterol, uric acid, pyruvic acid, etc.

Furthermore, although exemplified was a device wherein a test paper impregnated with a chromogenic reagent is used and the coloring of the test paper is measured as the reflectance ratio in the above example, the present invention is not limited thereto but rather can be applied to the calibration reagent used for calibrating a device wherein a blood sample is added to a solution containing a chromogenic reagent stored in a test tube and the like to measure the transmissive-absorbance of the liquid, for example. This is obvious from the fact that absorption spectrum was shown in the embodiment and example described above.

### Example 2

In this example, Alphazurine A and the dye A are used in combination to adjust the calibration reagent. Also, in a comparative example 2, Erioglaucine (Fig. 17), Alphazurine A (Fig. 18), or the dye A (Fig. 19) were used independently to adjust calibration reagents. Further, as a reference example, measurements were made on a blood sample (blood in which the glucose concentration is adjusted to 180 mg/dL).

First, as the comparative example 2, calibration reagents were prepared by adding Erioglaucine (0.072 % by mass), Alphazurine A (0.24 % by mass), or the dye A (0.45 % by mass) independently to pure water.

On the otherhand, as the example 2, a calibration reagent containing two dyes was prepared. More specifically, it is pure water added with Alphazurine A (0.08 % by mass) and the dye A (0.32 % by mass).

Further, glucose (made by Kanto Chemical Co., Inc.) was added to both the calibration reagent samples of comparative example 2 and example 2 in such a way that their reflection-absorbances become similar (approximately 600) to the refection-absorbance of the blood sample at the wavelength of 610 nm. In addition, 0.72 % by mass of viscosity adjusting agent (carboxymethylcellulose (CMC 1260, made by Daicel Chemical Industries, Ltd.)), 250 mM of pH buffering agent (piperazine-1, 4-bis (2-ethanesulfonic acid) (PIPES, made by Dojindo Laboratories)), and 0.05 % by mass of preservative agent (sodium azide, made by Kanto Chemical Co. , Inc.) are added as additives other than glucose.

Coloring tests were then conducted for the example 2, comparative example 2, and the reference example (blood sample). More specifically, the reflectance ratio was measured at various temperatures (10, 15, 20, 25 and 30°C) by use of a spectrometer USB-2000 (Pj-1181/Ocean Optics) when sufficient coloring is obtained by spotting each sample on the test tool (Medisafe Tip made by Terumo Corporation) holding a test paper impregnated with the chromogenic reagent (a mixture of glucose oxydase (GOD), peroxidase (POD), and 4-aminoantipyrine, and N-ethyl-N-(2-hydroxy-3-sulfopropyl)-m-toluidine) (e.g., 10 seconds after dropping the samples). The reflection-absorbance is obtained from this reflectance and is a value calculated as 256/reflectance. Therefore, the value of resultant reflection-absorbance includes, in addition to the absorbance caused by the dye added to the calibration reagent, the absorbance caused by the pigment, which is generated by the reaction between the chromogenic reagent impregnating in the test paper and glucose. This measurement is repeated five times in total in a similar way, and the average is accepted as the reflection-absorbance value. The value of 256/reflectance is arbitrarily and conveniently defined to obtain absorbance from reflectance and it is not limited thereto. The result will be described below with reference to the accompanying drawings.

As described above, Fig. 15 is a graph showing the reflection-absorbance spectra at various temperatures for the reference example (blood sample) overlapping each other. As shown in Fig. 15, the reflection-absorbance of the blood sample tends to shift generally in the direction of increase of the absorbance as the measurement temperature increases.

Additionally, as mentioned before, Figs. 17 - 19 are the graphs showing the results of reflection-absorbance spectrum measurements in comparative example 2. Among the dyes used here, one other than the dye A can be found on the Sigma-Aldrich Handbook of Stains, Dyes and Indicators, Aldrich Chemical Company, 1990.

As can be seen obviously from the comparison between Figs. 17 - 19 (comparative example 2) and Fig. 15 (reference example), there is a marked difference between their temperature characteristics at the wavelength of 610 nm. Also, the inclination of the graph plotted on a coordinate system having temperature on the horizontal axis and reflection-absorbance (610 nm) on the vertical axis is smaller for a case added with Alphazurine A and larger in case of the dye A compared with blood as shown in Fig. 21. As a result, if it is tried to compensate the fluctuations of the measurement values depending on temperature by applying the temperature compensation algorithm for blood to the calibration reagent described above, the calibration reagent of a single dye cannot compensate the value as in the case of the blood sample, and the measurement value causes departure along with the measurement temperature change as shown in Fig. 22. Fig. 22 is a graph showing the measurement value obtained by executing calibration with the calibration reagent sample of comparative example 2 and example 2 as well as the blood sample of the reference example, and then applying the temperature compensation algorithm for blood. In applying the temperature compensation algorithm, measurement was conducted by using a commercial blood glucose meter (Medisafe Mini GR-102 and Medisafe Tip made by Terumo Corporation) and placing the blood glucose meter and the calibration reagents or the blood sample in an environmental tester adjusted to specific temperatures (10, 15, 20, 25 and 30°C). The measurement was conducted 10 times in total and the average was accepted as the measurement value. Further, the hematocrit compensation (Ht compensation) was also conducted for the blood sample.

In the meanwhile, the evaluation of the measurement value was conducted by executing similar coloring tests and applying the temperature compensation algorithm for the calibration reagent samples prepared in example 2. Fig. 20 is a graph showing the reflection-absorbance spectra at various temperatures for the calibration reagent samples of example 2 overlapping each other. Fig. 21 shows a graph where the reflection-absorbance at the wavelength of 610 nm is plotted against temperature. Fig. 22 shows a graph of the measurement value obtained by conducting a calibration with the calibration reagent sample of example 2 (a combination of Alphazurine A and the dye A) and applying a temperature compensation algorithm for blood similar to the one discussed above.

As shown in Fig. 21, according to the calibration reagent sample of example 2, the variation tendency of the reflection-absorbance depending on the measurement temperature, obtained by spotting the calibration reagent on one surface of the test paper provided on the blood component measurement device and measuring the reflection-absorbance by irradiating the other surface of the test paper with a light of the specified wavelength, becomes similar to the variation tendency of the reflection-absorbance depending on the measurement temperature measured by spotting blood instead of said calibration reagent.

Also, it is learned from the comparison between Fig. 15 and Fig. 20 that the reflection-absorbance spectrum of the calibration reagent sample of example 2 shows a tendency quite similar to the inclination tendency of the spectrum of the reference example (blood sample) in the wavelength range of approximately 580 - 630 nm (especially 600 - 615 nm) that includes the wavelength of 610 nm characteristic to the generated pigment used for measuring glucose concentration under any temperature condition. Therefore, the calibration reagent sample of example 2 provides an advantage that it can compensate for the measurement error, due to the fact that the calibration reagent lacks blood pigment.

The results of these examples indicate that the present invention can provide a method of adjusting calibration reagents for accurately calibrating a measurement device even when the temperature condition of measurement fluctuates, a calibration reagent, and a calibration method for the measurement device using thereof. Moreover, it is shown that the color of the calibration reagent can become similar to the blood color sufficiently within the range of fluctuations of the wavelength used in the measurement according to a preferable example.

### Evaluation of spreading

In the coloring test of example 2 as described in the above, observed was the value including the contribution of the absorbance caused by pigment generated by the reaction between the chromogenic reagent and glucose in addition to the absorbance caused by the separately added dyes. The following test was conducted in order to eliminate such effects and to observe only the performance of the separately added dyes. In this test, the time course of the reflection-absorbance was observed while varying the measurement temperature in aqueous solutions containing Erioglaucine and Alphazurine A independently.

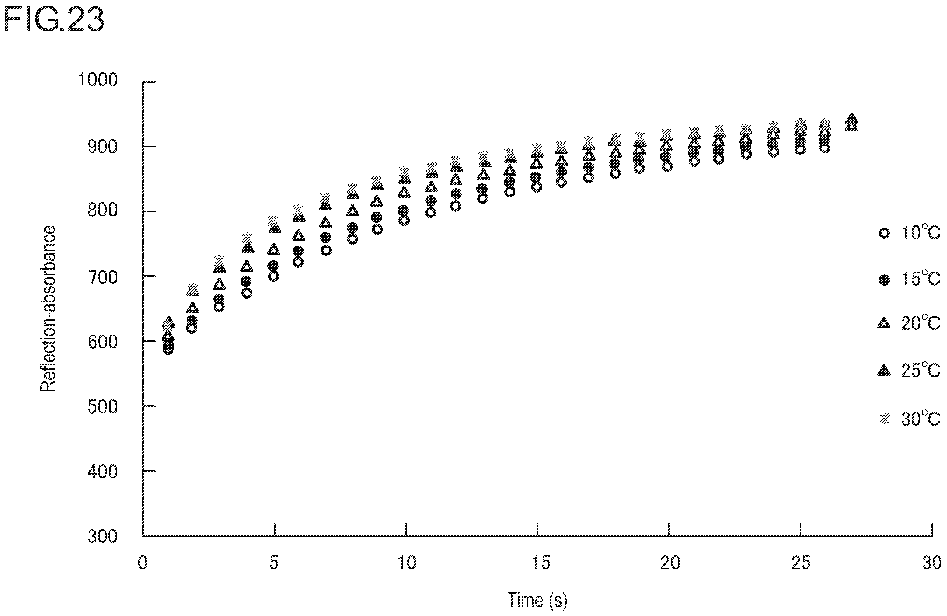

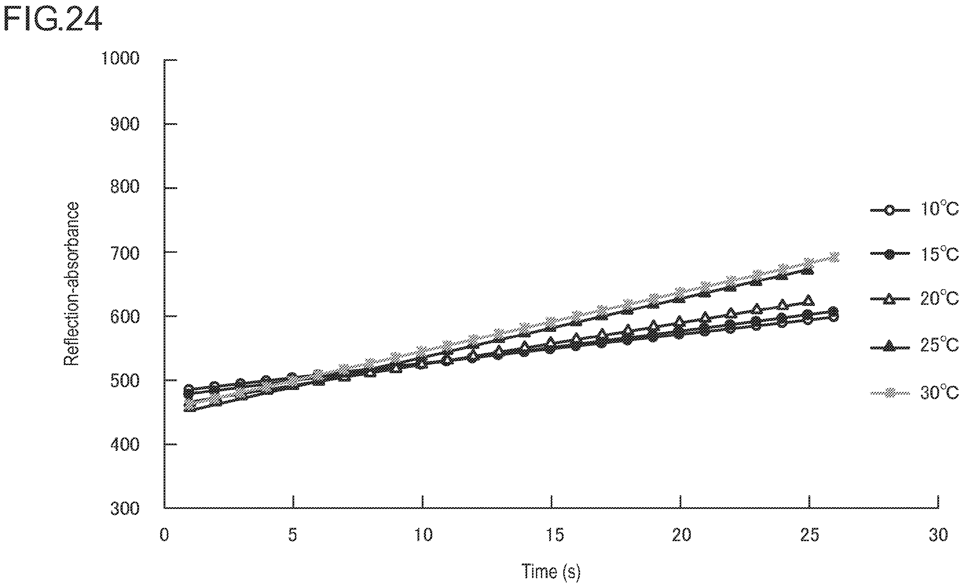

A test tool having a polyethersulfone (PES) membrane which is not impregnated with the chromogenic reagent (Medisafe Tip (made by Terumo Corporation) without containing the chromogenic reagent) was prepared. Also, an aqueous solution containing 0.072 % by mass of Erioglaucine and an aqueous solution containing 0.24 % by mass of Alphazurine A were prepared as samples for evaluation. Each sample was spotted on the PES membrane of the test tool prepared as above to start timing simultaneously, and the reflection-absorbance measurement was conducted by using the same technique described above for 26 - 27 seconds from the spotting at one second interval. Similar tests were repeated 10 times each at temperatures of 10, 15, 20, 25 and 30°C and thus obtained averages were recorded as the measurement values. The result is shown in Fig. 23 (Erioglaucine) and Fig. 24 (Alphazurine A). As shown in Fig. 23, the reflection-absorbance of Erioglaucine increases logarithmically with time (in other words, the rate of increase gradually decreases). On the other hand, the reflection-absorbance of Alphazurine A increases linearly with time. Moreover, in case of Erioglaucine, the higher the measurement temperature was, the larger the reflection-absorbance was at all points during the time course, but, in case of Alphazurine A, the higher the measurement temperature was, the smaller the reflection-absorbance was in the early period of measurement, while the value of reflection-absorbance increased with increasing temperature as the time went on. From this we learn that the time course of reflection-absorbance and the temperature sensitivity thereof vary with the type of dye. Although it is not certain why these differences exist, the following mechanism is suspected. For instance, it is estimated that Erioglaucine which has three sulfonic acid groups has a higher water solubility than Alphazurine A which has only two sulfonic acid groups. In the meanwhile, it is estimated that Erioglaucine is less absorbent to the PES membrane and moves relatively freely in the membrane. Since the mobility of molecules depends on the temperature, Erioglaucine that moves relatively free is susceptive to temperature changes, so that it is more temperature sensitive. On the other hand, Alphazurine A moves more slowly in the membrane so that it is less sensitive to temperature changes. Consequently, it is less temperature sensitive, i.e., has a better temperature characteristic.

The present application is based on Japanese Patent Application No. 2008-090401 filed on March 31, 2008 and Japanese Patent Application No. 2008-220493 filed on August 28, 2008.

## Claims

1. A method of adjusting a calibration reagent used in calibrating a blood component measurement device intended for optical measurement of a blood component using a test paper (101), the method comprising:
combining two (first and second) or more kinds of different dyes and adjusting in such a way that the variation tendency of the reflection-absorbance depending on the measurement temperature when measured by spotting said calibration reagent on one surface of said test paper (101) and irradiating the other surface with a light of a specified wavelength becomes similar to the variation tendency of the reflection-absorbance depending on the measurement temperature when measured by spotting blood instead of said calibration reagent, **characterized in that**
said two or more kinds of dyes comprise 6-amino-4-hydroxy-5-((4-nitro-2-sulfophenyl)azo)-2-naphthalenesulfonic acid and Alphazurine A.

2. The adjustment method according to claim 1, wherein said two or more kinds of dyes are combined in such a way as to compensate the measurement error resulting from the fact that the calibration reagent does not contain the pigment component in blood.

3. The adjustment method according to claim 2 **characterized in**, placing the center value of the FWHM of the emission spectrum of the light emitting element (102) at the nominal wavelength of said light emitting element (102) used in said blood component measurement device, selecting and combining two or more kind of dyes having different inclinations of reflection-absorbance spectrum within the wavelength range of at least said FWHM having its center value at said nominal wavelength, and adjusting the calibration reagent in such a way that the resultant inclination tendency of the reflection-absorbance spectrum within said wavelength range becomes similar to that of the reflection-absorbance spectrum of blood within said wavelength range, and/or
wherein said two or more kinds of dyes comprise one or more kinds of dyes selected from a group of dyes having a maximum absorption wavelength on a wavelength side shorter than 610 nm, and one or more kinds of dyes selected from a group of dyes having a maximum absorption wavelength on a wavelength side longer than 610 nm.

4. The adjustment method according to claim 1, **characterized in**:
combining said first and second dyes having different inclinations of absorption spectrum within a wavelength range of at least a full width at half maximum ("FWHM") of an emission spectrum having its center value at the nominal wavelength of a light emitting element (102) used in said blood component measurement device; and
adjusting the calibration reagent in such a way that the shape of said combined absorption spectrum within said wavelength range becomes similar to the shape of the absorption spectrum of blood within said wavelength range.

5. A calibration reagent used in calibrating a blood component measurement device intended for optical measurement of a blood component using a test paper (101), wherein:
the calibration reagent comprises a known amount of the blood component, and two (first and second) or more kinds of different dyes combined in such a way that the variation tendency of the reflection-absorbance depending on the measurement temperature when measured by spotting said calibration reagent on one surface of the test paper (101) and irradiating the other surface of the test paper (101) with a light of the specified wavelength, becomes similar to the variation tendency of the reflection-absorbance depending on the measurement temperature when measured by spotting blood instead of said calibration reagent, **characterized in that**
said two or more kinds of dyes comprise 6-amino-4-hydroxy-5-((4-nitro-2-sulfophenyl)azo)-2-naphthalenesulfonic acid and Alphazurine A.

6. A calibration reagent according to claim 5, **characterized in**:
said first and second dyes having different inclinations of absorption spectrum within a wavelength range of at least a full width at half maximum ("FWHM") of an emission spectrum having its center value at the nominal wavelength of a light emitting element (102) used in said blood component measurement device; and
said first and second dyes are combined in such a way that the shape of said combined absorption spectrum within said wavelength range becomes similar to the shape of the absorption spectrum of blood within said wavelength range.

7. The calibration reagent according to claim 5 or 6, wherein the wavelength used in measuring said blood component is the wavelength based on the chromogenic reagent that reacts with said blood component.

8. A method of calibrating a blood component measurement device intended for optical measurement of a blood component using a test paper (101) **characterized in**:
using the calibration reagent described in any one of claims 5 to 7, obtaining a measurement value by applying said calibration reagent to said blood component measurement device, and determining whether or not said measurement value is within the allowable range set up from the known component amount of said calibration reagent.

## Patentansprüche

1. Verfahren zum Einstellen eines Kalibrationsreagenzes, das beim Kalibrieren einer Blutkomponentenmessvorrichtung verwendet wird, die zur optischen Messung einer Blutkomponente unter Verwendung eines Testpapiers (101) bestimmt ist, wobei das Verfahren umfasst:
Kombinieren von zwei (ersten und zweiten) oder mehr Arten von unterschiedlichen Farbstoffen und Einstellen in solch einer Weise, dass die Variationstendenz des Reflexions-Absorptionsgrads, die von der Messtemperatur abhängig ist, wenn durch Spotten des Kalibrationsreagenzes auf eine Oberfläche des Testpapiers (101) und Bestrahlen der anderen Oberfläche mit einem Licht einer bestimmten Wellenlänge gemessen wird, ähnlich wird zu der Variationstendenz des Reflexions-Absorptionsgrads, die von der Messtemperatur abhängig ist, wenn durch Spotten des Bluts anstelle des Kalibrationsreagenzes gemessen wird, **dadurch gekennzeichnet, dass**
die zwei oder mehr Arten an Farbstoffen 6-Amino-4-hydroxy-5-((4-nitro-2-sulfophenyl)azo)-2-naphthalensulfonsäure und Alphazurin A umfassen.

2. Verfahren zum Einstellen nach Anspruch 1, wobei die zwei oder mehr Arten an Farbstoffen in solch einer Art kombiniert werden, um den Messfehler zu kompensieren, der von der Tatsache resultiert, dass das Kalibrationsreagenz die Pigmentkomponente im Blut nicht enthält.

3. Verfahren zum Einstellen nach Anspruch 2, **gekennzeichnet durch**, Platzieren des Mittelpunktwerts der Halbwertsbreite des Emissionsspektrums des Licht-emittierenden Elements (102) bei der Nominalwellenlänge des Licht-emittierenden Elements (102), das in der Blutkomponentenmessvorrichtung verwendet wird, Auswählen und Kombinieren von zwei oder mehr Arten von Farbstoffen mit unterschiedlichen Neigungen des Reflexions-Absorptionsgrad-Spektrums innerhalb des Wellenlängenbereichs von zumindest der Halbwertsbreit mit dessen Mittelpunktswert bei der Nominalwellenlänge, und Einstellen des Kalibrationsreagenzes in solch einer Weise, dass die resultierende Neigungstendenz des Reflexions-Absorptionsgrad-Spektrums innerhalb des Wellenlängenbereichs ähnlich wird zu der des Reflexions-Absorptionsgrad-Spektrums von Blut innerhalb des Wellenlängenbereichs, und/oder
wobei die zwei oder mehr Arten von Farbstoffen eine oder mehr Arten von Farbstoffen, ausgewählt aus der Gruppe von Farbstoffen mit einer maximalen Absorptionswellenlänge auf einer Wellenlängenseite kürzer als 610 nm, und eine oder mehr Arten von Farbstoffen, ausgewählt aus einer Gruppe von Farbstoffen mit einer maximalen Absorptionswellenlänge auf einer Wellenlängenseite länger als 610 nm, umfassen.

4. Verfahren zum Einstellen nach Anspruch 1, **gekennzeichnet durch**:
Kombinieren der ersten und zweiten Farbstoffe mit unterschiedlichen Neigungen des Absorptionsspektrums innerhalb eines Wellenlängenbereichs von zumindest einer Halbwertsbreite "FWHM") eines Emissionsspektrums mit dessen Mittelpunktswert bei der Nominalwellenlänge eines Licht-emittierenden Elements (102), das in der Blutkomponentenmessvorrichtung verwendet wird; und
Einstellen des Kalibrationsreagenzes in solch einer Weise, dass die Form des kombinierten Absorptionsspektrums innerhalb des Wellenlängenbereichs ähnlich zu der Form des Absorptionsspektrums von Blut innerhalb des Wellenlängenbereichs wird.

5. Kalibrationsreagenz, das beim Kalibrieren einer Blutkomponentenmessvorrichtung verwendet wird, die zum optischen Messen einer Blutkomponente unter Verwendung eines Testpapiers (101) bestimmt ist, wobei:
das Kalibrationsreagenz eine bekannte Menge der Blutkomponente umfasst, und zwei (erste und zweite) oder mehr Arten an unterschiedlichen Farbstoffen, die in solch einer Weise kombiniert sind, dass die Variationstendenz des Reflexions-Absorptionsgrads, die von der Messtemperatur abhängig ist, wenn durch Spotten des Kalibrationsreagenzes auf eine Oberfläche des Testpapiers (101) und Bestrahlen der anderen Oberfläche mit einem Licht einer bestimmten Wellenlänge gemessen wird, ähnlich wird zu der Variationstendenz des Reflexions-Absorptionsgrads, die von der Messtemperatur abhängig ist, wenn durch Spotten des Bluts anstelle des Kalibrationsreagenzes gemessen wird, **dadurch gekennzeichnet, dass**
die zwei oder mehr Arten an Farbstoffen 6-Amino-4-hydroxy-5-((4-nitro-2-sulfophenyl)azo)-2-naphthalensulfonsäure und Alphazurin A umfassen.

6. Kalibrationsreagenz nach Anspruch 5, **gekennzeichnet durch**:
die ersten und zweiten Farbstoffe mit unterschiedlichen Neigungen des Absorptionsspektrums innerhalb eines Wellenlängenbereichs von zumindest einer Halbwertsbreite "FWHM") eines Emissionsspektrums mit dessen Mittelpunktswert bei der Nominalwellenlänge eines Licht-emittierenden Elements (102), das in der Blutkomponentenmessvorrichtung verwendet wird; und
dass die ersten und zweiten Farbstoffe in solch einer Weise kombiniert werden, dass die Form des kombinierten Absorptionsspektrums innerhalb des Wellenlängenbereichs ähnlich wird zu der Form des Absorptionsspektrums von Blut innerhalb des Wellenlängenbereichs.

7. Kalibrationsreagenz nach Anspruch 5 oder 6, wobei die beim Messen der Blutkomponente verwendete Wellenlänge die Wellenlänge basierend auf dem chromogenen Reagens ist, das mit der Blutkomponente reagiert.

8. Verfahren zum Kalibrieren einer Blutkomponentenmessvorrichtung, die zur optischen Messung einer Blutkomponente unter Verwendung eines Testpapiers (101) bestimmt ist, **gekennzeichnet durch**:
Verwenden des Kalibrationsreagenzes, das in einem der Ansprüche 5 bis 7 beschrieben ist, Erhalten eines Messwerts durch Aufbringen des Kalibrationsreagenzes zu der Blutkomponentenmessvorrichtung, und Bestimmen, ob der Messwert innerhalb des erlaubten Bereichs ist oder nicht, der von der bekannten Komponentenmenge des Kalibrationsreagenzes festgelegt wird.

## Revendications

1. Procédé d'ajustement d'un réactif d'étalonnage utilisé dans l'étalonnage d'un dispositif de mesure des composants du sang destiné à mesurer optiquement un composant du sang à l'aide d'un papier réactif (101), le procédé comprenant les étapes consistant à :
combiner deux (premier et second) types ou plus de différents colorants et procéder à un ajustement de telle sorte que la tendance de variation de l'absorbance de réflexion en fonction de la température de mesure mesurée en déposant ledit réactif d'étalonnage sur une surface dudit papier réactif (101) et en irradiant l'autre surface avec une lumière d'une longueur d'onde spécifiée devient similaire à la tendance de variation de l'absorbance de réflexion en fonction de la température de mesure mesurée en déposant du sang à la place dudit réactif d'étalonnage, **caractérisé en ce que**
lesdits deux types ou plus de colorants comprennent 6-amino-4-hydroxy-5-((4-nitro-2-sulfophényl)azo)-2-acide naphtalènesulfonique et alphazurine A.

2. Procédé d'ajustement selon la revendication 1, dans lequel lesdits deux types ou plus de colorants sont combinés de manière à compenser l'erreur de mesure provenant du fait que le réactif d'étalonnage ne contient pas le composant pigmentaire du sang.

3. Procédé d'ajustement selon la revendication 2, **caractérisé par** les étapes consistant à placer la valeur centrale de la largeur à mi-hauteur (« FWHM ») du spectre d'émission de l'élément émetteur de lumière (102) à la longueur d'onde nominale dudit élément émetteur de lumière (102) utilisé dans ledit dispositif de mesure des composants du sang, sélectionner et combiner deux types ou plus de colorants ayant différentes inclinaisons du spectre d'absorbance de réflexion à l'intérieur de la gamme de longueurs d'onde d'au moins la largeur à mi-hauteur (« FWHM ») dont la valeur centrale est à ladite longueur d'onde nominale, et ajuster le réactif d'étalonnage de telle sorte que la tendance d'inclinaison résultante du spectre d'absorbance de réflexion à l'intérieur de ladite gamme de longueurs d'onde deviennent similaire à celle du spectre d'absorbance de réflexion du sang à l'intérieur de ladite gamme de longueurs d'onde et/ou,
dans lequel lesdits deux types ou plus de colorants comprennent un ou plusieurs types de colorants sélectionnés parmi un groupe de colorants ayant une longueur d'onde d'absorption maximale sur un côté de longueur d'onde inférieure à 610 nm, et un ou plusieurs types de colorants sélectionnés parmi un groupe de colorants ayant une longueur d'onde d'absorption maximale sur un côté de longueur d'onde supérieure à 610 nm.

4. Procédé d'ajustement selon la revendication 1, **caractérisé par** les étapes consistant à :
combiner lesdits premier et second colorants ayant différentes inclinaisons du spectre d'absorption à l'intérieur d'une gamme de longueurs d'onde d'au moins la largeur à mi-hauteur (« FWHM ») d'un spectre d'émission dont la valeur centrale à la longueur d'onde nominale d'un élément émetteur de lumière (102) à la longueur d'onde nominale dudit élément émetteur de lumière (102) utilisé dans ledit dispositif de mesure des composants du sang ; et
ajuster le réactif d'étalonnage de telle sorte que la forme dudit spectre d'absorption combiné à l'intérieur de ladite gamme de longueurs d'onde devienne similaire à la forme du spectre d'absorption du sang à l'intérieur de ladite gamme de longueurs d'onde.

5. Réactif d'étalonnage utilisé dans l'étalonnage d'un dispositif de mesure des composants du sang destiné à mesurer optiquement un composant du sang à l'aide d'un papier réactif (101), dans lequel :
le réactif d'étalonnage comprend une quantité connue du composant du sang, et deux (premier et second) types ou plus de différents colorants combinés de telle sorte que la tendance de variation de l'absorbance de réflexion en fonction de la température de mesure mesurée en déposant ledit réactif d'étalonnage sur une surface du papier réactif (101) et en irradiant l'autre surface du papier réactif (101) avec une lumière ayant la longueur d'onde spécifiée, devienne similaire à la tendance de variation de l'absorbance de réflexion en fonction de la température de mesure mesurée en déposant du sang à la place dudit réactif d'étalonnage, **caractérisé en ce que**
lesdits deux types ou plus de colorants comprennent 6-amino-4-hydroxy-5-((4-nitro-2-sulfophényl)azo)-2-acide naphtalènesulfonique et alphazurine A.

6. Réactif d'étalonnage selon la revendication 5, **caractérisé en ce que** :
lesdits premier et second colorants ayant différentes inclinaisons du spectre d'absorption à l'intérieur d'une gamme de longueurs d'onde d'au moins la largeur à mi-hauteur (« FWHM ») d'un spectre d'émission dont la valeur centrale à la longueur d'onde nominale d'un élément émetteur de lumière (102) utilisé dans ledit dispositif de mesure des composants du sang ; et
lesdits premier et second colorants sont combinés de telle sorte que la forme dudit spectre d'absorption combiné à l'intérieur de ladite gamme de longueurs d'onde devienne similaire à la forme du spectre d'absorption du sang à l'intérieur de ladite gamme de longueurs d'onde.

7. Réactif d'étalonnage selon la revendication 5 ou 6, dans lequel la longueur d'onde utilisée pour mesurer ledit composant du sang est la longueur d'onde basée sur le réactif chromogène qui réagit avec ledit composant du sang.

8. Procédé d'étalonnage d'un dispositif de mesure des composants du sang destiné à mesurer optiquement un composant du sang à l'aide d'un papier réactif (101), **caractérisé par** les étapes consistant à :
utiliser le réactif d'étalonnage décrit dans l'une quelconque des revendications 5 à 7, obtenir une valeur de mesure en appliquant ledit réactif d'étalonnage sur ledit dispositif de mesure des composants du sang, et déterminer si ladite valeur de mesure est comprise ou non dans la plage admissible fixée à partir de la quantité de composant connue dudit réactif d'étalonnage.
